# EUROPÄISCHE PATENTSCHRIFT

(11) **EP 1 246 503 B1**
(45) Veröffentlichungstag und Bekanntmachung des Hinweises auf die Patenterteilung: **09.02.2005**
(21) Anmeldenummer: 02004438.4
(22) Anmeldetag: 26.02.2002
(51) Int. Cl.: H04R 25/00, A61N 1/36, A61F 11/04

(54) **Vollständig implantierbares Hörsystem**
Completely implantable hearing system
Système auditif entièrement implantable

(30) Priorität: 26.03.2001 DE 10114838
(43) Veröffentlichungstag der Anmeldung: 02.10.2002
(73) Patentinhaber: Cochlear Limited, Lane Cove, NSW 2066 (AU)
(72) Erfinder: Leysieffer, Hans, Dr.-Ing., 82024 Taufkirchen (DE); Baumann, Joachim W., Dipl.-Ing., 85570 Markt Schwaben (DE)
(74) Vertreter: Schwan - Schwan - Schorer

(56) Entgegenhaltungen:
- EP-A- 0 683 621
- EP-A- 0 831 674
- EP-A- 1 043 914
- WO-A-00/69215
- US-A- 6 077 215

## Beschreibung

Die Erfindung betrifft ein vollständig implantierbares Hörsystem zur Rehabilitation von Hörstörungen mit mindestens einem Sensor, der mindestens Luftschall aufnimmt und in elektrische Signale umwandelt, einem Elektronikmodul mit einer elektronischen Anordnung zur Audiosignalverarbeitung und -verstärkung, einer ausgangsseitigen aktorischen Anordnung zum Stimulieren des Mittel- oder Innenohres sowie einer elektrischen Energieversorgungseinheit.

Unter dem Begriff "Hörstörung" sollen vorliegend alle Arten von Innenohrschäden bis hin zur vollständigen postlingualen Ertaubung oder prälingualen Gehörlosigkeit, kombinierte Innen- und Mittelohrschäden sowie auch zeitweise auftretende oder permanente Ohrgeräusche (Tinnitus) verstanden werden.

Die Rehabilitation sensorischer Hörstörungen mit teilimplantierbaren, elektronischen Systemen hat in den letzten Jahren einen bedeutenden Stellenwert erhalten. Insbesondere gilt dies für den Patientenkreis, bei dem das Gehör durch Unfall, Krankheit oder sonstige Einflüsse vollständig ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hörnerv stimuliert und somit ein Höreindruck erzeugt werden, der bis zu einem offenen Sprachverständnis führen kann. Bei diesen sogenannten Cochlea Implantaten wird ein Reizelektroden-Array in die Cochlea eingeführt, das von einem elektronischen System angesteuert wird, das als hermetisch dichtes und biokompatibel eingekapseltes Elektronikmodul im knöchernen Bereich hinter dem Ohr (Mastoid) operativ eingebettet ist. Das elektronische System enthält jedoch im wesentlichen nur Dekodier- und Treiberschaltungen für die Reizelektroden. Die akustische Schallaufnahme, die Wandlung dieses Schallsignals in elektrische Signale und deren weitere Verarbeitung erfolgt grundsätzlich extern in einem sogenannten Sprachprozessor, der außen am Körper getragen wird. Der Sprachprozessor setzt die vorverarbeiteten Signale entsprechend kodiert auf ein hochfrequentes Trägersignal um, das über eine induktive Kopplung durch die geschlossene Haut (transkutan) zu dem Implantat übertragen wird. Das schallaufnehmende Mikrofon befindet sich ausnahmslos außerhalb des Körpers und in den meisten Anwendungen in einem an der Ohrmuschel getragenen Gehäuse eines Hinter-dem-Ohr-Hörgerätes (HdO), und es ist mittels eines Kabels mit dem Sprachprozessor verbunden.

Neben der Rehabilitation gehörloser beziehungsweise ertaubter Patienten mit Cochlea Implantaten existieren seit geraumer Zeit Ansätze, Patienten mit einer sensorineuralen Hörstörung, die operativ nicht behebbar ist, mit teil- beziehungsweise vollimplantierbaren Hörgeräten eine bessere Rehabilitation als mit konventionellen Hörgeräten zu bieten. Das Prinzip besteht in den überwiegenden Ausführungsformen darin, ein Ossikel des Mittelohres oder das Innenohr direkt über einen mechanischen beziehungsweise hydromechanischen Reiz zu stimulieren und nicht über das verstärkte akustische Signal eines konventionellen Hörgerätes, bei dem das verstärkte Schallsignal dem äußeren Gehörgang zugeführt wird. Der aktorische Stimulus dieser elektromechanischen Systeme wird mit verschiedenen physikalischen Wandlerprinzipien realisiert wie zum Beispiel durch elektromagnetische und piezoelektrische Systeme. Der Vorteil dieser Verfahren wird hauptsächlich in der gegenüber konventionellen Hörgeräten verbesserten Klangqualität und bei vollimplantierten Systemen in der Unsichtbarkeit der Hörprothese gesehen. Solche teil- und vollimplantierbaren elektromechanischen Hörgeräte sind beispielhaft von H.P. Zenner et al (HNO 1998, 46:844-852), H. Leysieffer et al. ("Ein vollständig implantierbares Hörsystem für Innenohrschwerhörige: TICA LZ 3001", HNO 46:853-863, 1998) und H.P. Zenner et al. ("Totally implantable hearing device for sensorineural hearing loss", The Lancet, Vol. 352, Nov. 1998, No. 9142, Seite 1751) beschrieben.

Viele Patienten mit einem Innenohrschaden leiden zusätzlich unter zeitweise auftretenden oder permanenten Ohrgeräuschen (Tinnitus), die operativ nicht behebbar sind und gegen die bis heute keine zugelassenen medikamentösen Behandlungsformen existieren. Daher sind sogenannte Tinnitus-Maskierer erhältlich; dies sind kleine, batteriebetriebene Geräte, die ähnlich einem Hörgerät hinter oder im Ohr getragen werden und durch artifizielle Schalle, die über einen zum Beispiel Hörgeräte-Lautsprecher in den Gehörgang abgestrahlt werden, den Tinnitus auf psychoakustisch wirkende Weise verdecken ("maskieren") und das störende Ohrgeräusch so möglichst unter die Wahrnehmungsschwelle absenken. Die artifiziellen Schalle sind häufig Schmalbandgeräusche (zum Beispiel Terzrauschen), die in ihrer spektralen Lage und Lautstärkepegel über ein Programmiergerät einstellbar sind, um eine möglichst optimale Anpassung an die individuelle Ohrgeräuschsituation zu ermöglichen. Darüberhinaus existiert seit kurzem die sogenannte "Retraining-Methode", wobei durch die Kombination eines mentalen Trainingsprogrammes und die Darbietung eines breitbandigen Schalles (Rauschen) nahe der Ruhehörschwelle die Wahrnehmbarkeit des Tinnitus ebenfalls weitgehend unterdrückt werden soll. Diese Geräte werden auch als "Noiser" bezeichnet.

Bei beiden oben genannten Methoden zur apparativen Therapie des Tinnitus sind hörgeräteähnliche, technische Geräte außen am Körper im Ohrbereich sichtbar mitzuführen, die den Träger stigmatisieren und daher nicht gerne getragen werden.

Seit kurzem sind teil- und vollimplantierbare Hörsysteme zur Rehabilitation eines Innenohrschadens in der klinischen Anwendung. Bei dem vollständig implantierbaren, elektromechanischen Hörsystem TICA® (H.P. Zenner et al. ("Totally implantable hearing device for sensorineural hearing loss", The Lancet, Vol. 352, Nov. 1998, No. 9142, Seite 1751) wird ein subkutan in der hinteren knöchernen Gehörgangswand eingesetzter Audioschallsensor (Mikrofon) verwendet (US-A-5 814 095 und US-A-5 999 632). Die ersten klinischen Erfahrungen mit diesem System zeigen, dass bei einigen Patienten die eigene Stimme sowie andere Körperschallschwingungen wie Kau- und Schluckgeräusche deutlich und auch störend laut wahrgenommen werden. Dies liegt daran, dass neben den von außen einfallenden Luftschallsignalen auch körperschallinduzierte Signale durch Knochenfortleitung auf den Audiosensor einwirken und über das implantierte Hörsystem ebenfalls verstärkt an das Innenohr weitergegeben werden. Durch diese Mischung der einzelnen Eingangssignalkomponenten verändert sich bei diesen Patienten das gewohnte und gewünscht natürliche Klangbild der Eigenstimme oder der verstärkte Körperschallanteil wirkt als Störgeräusch, das maskierend wirken kann, wodurch das Hören und Verstehen von externer Sprache erschwert wird. Gegen diesen Effekt kann mit den bekannten Hörimplantaten nichts unternommen werden, da auf den implantierten Audiosensor und dessen eingangsseitige Wirkung kein Einfluss genommen werden kann. Dieser störende Effekt ist auch bei zukünftig zu erwartenden vollständig implantierbaren Cochlea Implantaten anzunehmen, der die hier erforderliche Hörrehabilitation noch deutlicher beinträchtigen kann als bei elektromechanischen Implantaten für Schwerhörige. Ein vielleicht möglicher Ausweg wäre eine von Körperschall vollständig abgekoppelte Implantation des Audiosensors durch geeignete körperschallisoherende Implantatmaterialien und/oder konstruktive Maßnahmen im Audiosensor selbst. Hierdurch würde zumindest theoretisch der beschriebene negative Effekt eliminiert, jedoch fallen damit auch alle körperschallinduzierten Anteile der eigenen Stimme wie insbesondere die mechanischen Kehlkopfschwingungen weg, die zu einem gewohnt natürlichen Klangbild der eigenen Stimme unerlässlich sind. Zur Erhöhung der Akzeptanz vollimplantierbarer Hörsysteme gleich welcher Art der aktorischen Ausgangsstimuli ist daher eine technische Lösung wünschenswert, die individuell einerseits eine ungewollte Verstarkung von körperschallinduzierten Signalen verhindert und andererseits einen solchen Übertragungsanteil dieser Signale gestattet, dass ein angenehmer und natürlich Höreindruck insbesondere der eigenen Stimme gewährleistet ist.

Ausgehend von einem System der eingangs genannten Art wird eine Lösung für das vorstehend beschriebene Problem erfindungsgemäß dadurch erreicht, dass das Hörsystem mindestens einen weiteren implantierbaren Sensor aufweist, der mindestens körperschallinduzierte Signale aufnimmt und in elektrische Signale umwandelt, und dass durch elektronisches Verrechnen der Luftschallsensorsignale und der Körperschallsensorsignale in der elektronischen Anordnung das Verhältnis von Luft- und Körperschallsignalen individuell einstellbar ist. Dieses Verhältnis lässt sich insbesondere so einstellen, dass nach weiterer Signalverarbeitung und Fortleitung an die ausgangsseitige aktorische Anordnung ein ausgewogener Höreindruck zwischen externen Luftschallsignalen und körpereigenen Schallsignalen, insbesondere der eigenen Stimme, hervorgerufen wird.

Vorteilhafte weitere Ausgestaltungen der Erfindung ergeben sich aus den Unteransprüchen.

Der beziehungsweise die Körperschallsensoren sind bevorzugt im knöchernen Bereich des Schädels lokalisiert. Beispielhaft kann der Sensor separat vom Elektronikmodul des betreffenden Implantates angeordnet sein und mit diesem fest oder lösbar, zum Beispiel über eine lösbare Steckverbindung, verbunden sein. Ein vorteilhafter Implantationsort ist der knöcherne Bereich des Mastoids hinter dem Außenohr. Bei dieser Ausführungsform ist der Sensor in einem hermetisch dichten und an der Oberfläche biokompatibel gestalteten Gehäuse untergebracht, das zur besonders guten Körperschalleinkopplung beispielsweise direkt mit dem Knochen des Applikationsortes mechanisch fest, zum Beispiel durch Anschrauben, verbunden wird.

Der Körperschallsensor kann aber auch in dem gleichen Gehäuse untergebracht sein wie der Luftschallsensor, wenn dieser in mechanischem Kontakt zu knöchernen Bereichen des Schädels steht, wie beispielsweise das aus US-A-5 814 095 und US-A-5 999 632 bekannte Mikrofon, das zur subkutanen Implantation in der hinteren, knöchernen Gehörgangswand vorgesehen ist. Weiterhin ist eine vorteilhafte Applikation des beziehungsweise der Körperschallsensoren die Unterbringung im Gehäuse des Implantat-Elektronikmoduls selbst. Bei implantierbaren Hörsystemen wird dieses Gehäuse grundsätzlich im Knochen des Mastoids eingebettet, was eine gute Einkopplung von Körperschall erwarten lässt. Ein weiterer Vorteil der beiden letztgenannten Varianten ist, dass der Körperschallsensor dann nicht selbst hermetisch dicht und biokompatibel ausgestaltet sein muss wie bei der erstgenannten Variante einer separaten, externen Applikation in einem eigenen Gehäuse.

Der beziehungsweise die Körperschallsensoren können nach jedem bekannten elektromechanischen Wandlerprinzip arbeiten, insbesondere elektromagnetisch, elektrodynamisch, piezoelektrisch, magnetostriktiv oder dielektrisch (kapazitiv). Der Körperschallsensor kann in an sich bekannter Weise (EP-A-387 299 und EP-A-385 574) als On-Chip-Halbleiterwandler ausgebildet sein. Bevorzugt arbeitet der Körperschallsensor nach dem bekannten Prinzip eines Beschleunigungsaufnehmers, wobei ein mechano-elektrischer Wandler im Sensorgehäuse an eine schwingfähig aufgehängte träge (seismische) Masse angekoppelt ist. Als mechano-elektrischer Wandler wird bevorzugt ein piezoelektrisches Element verwendet. Ein Körperschallsensor in Form eines On-Chip-Halbleiterwandlers kann vorteilhaft in einen Halbleiterbaustein des Elektronikmoduls oder eines Luftschall-Sensormoduls integriert sein. Weiterhin können auch handelsübliche, körperschallempfindliche kapazitive Elektretmikrofone der konventionellen Hörgeräteindustrie verwendet werden. Die letztgenannten Varianten haben den Vorteil, dass sie miniaturisiert aufgebaut werden können, was den Einbau des Hörimplantats im Kopfbereich begünstigt.

Der spektrale Übertragungsbereich der Körperschallsensoren liegt im Audiobereich, bevorzugt zwischen 100 Hz bis 10 kHz. Die Sensoren sind vorzugsweise hochabgestimmt, das heißt, die erste mechanische Resonanzfrequenz liegt am oberen Ende des angestrebten Übertragungsfrequenzbereiches. Dadurch wird erreicht, dass der Wandlungsfrequenzgang der Sensoren im Übertragungsbereich weitgehend frei von Resonanzen ist und damit eine möglichst geringe Welligkeit aufweist.

Das Einstellen des Verhältnisses von Luft- und Körperschallsignalen kann grundsätzlich in beliebiger Weise erfolgen, beispielsweise unter Verwendung eines Telemetriesystems, mittels dessen Daten zwischen einer externen Einheit und dem Implantat übertragen werden können, oder bei einem hochintelligenten Implantatsystem durch Reaktion des Implantats auf entsprechende Sprachsignale des Implantatträgers.

Die elektronische Weiterverarbeitung der Luft- und Körperschallsensorsignale erfolgt zweckmäßig über analoge Vorverstärker und eine analoge oder - nach entsprechender Analog-Digital-Wandlung - digitale Signalverarbeitung, wobei in dem Signalverarbeitungsmodul die Verrechnung der Sensorsignale derart erfolgt, dass sowohl Amplituden- wie auch Phasensignale berücksichtigt werden, um so eventuell notwendige Korrekturen verschiedener Phasen- und Gruppenlaufzeiten der Sensorsignale durchführen zu können. Diese Signalbearbeitung kann nach entsprechender spektraler Filterung auch in mehreren Frequenzbändern erfolgen. Die entsprechenden Signalverarbeitungsparameter sind im Implantat elektronisch speicherbar und können vorteilhaft über eine Telemetrieschnittstelle des Implantates von außen angepasst werden, um so beim individuellen Patienten nach Implantation und iterativer Programmierung dieser Parameter nach der Einheilphase und erster Hörerfahrung ein optimales Versorgungsergebnis zu erzielen.

Vorzugsweise erfolgt die Sensorsignalverarbeitung rein digital in einem digitalen Signalprozessor, dessen Softwarebetriebssystem beziehungsweise Teile davon telemetrisch nachgeladen beziehungsweise verändert werden können (DE-C-199 15 846), um so bei fortschreitenden wissenschaftlichen Erkenntnissen beziehungsweise Felderfahrungen den Patienten immer einen optimalen Signalverarbeitungsalgorithmus anbieten zu können, ohne dass das Implantat chirurgisch ausgetauscht werden muss.

Bevorzugte Ausführungsbeispiele der erfindungsgemäßen Anordnung sind nachstehend unter Bezugnahme auf die beiliegenden Zeichnungen näher beschrieben. Es zeigen:
- Fig. 1: ein Blockschaltbild eines vollimplantierbaren Hörsystems zur Rehabilitation einer Mittel- und/oder Innenohrstörung und/oder eines Tinnitus mit analoger Signalverarbeitung,
- Fig. 2: ein Blockschaltbild eines vollimplantierbaren Hörsystems zur Rehabilitation einer Mittel- und/oder Innenohrstörung und/oder eines Tinnitus mit digitaler Signalverarbeitung,
- Fig. 3: ein Blockschaltbild einer weiteren Ausführungsform eines vollimplantierbaren Hörsystems zur Rehabilitation einer Mittel- und/oder Innenohrstörung und/oder eines Tinnitus mit digitaler Signalverarbeitung,
- Fig. 4: eine schematische Schnittdarstellung eines vorliegend geeigneten Körperschallsensors,
- Fign. 5 bis 7: Ausführungsbeispiele von vollimplantierbaren Hörsystemen gemäß vorliegender Erfindung im implantierten Zustand mit unterschiedlicher Anordnung des Körperschallsensors und mit einkanaliger elektromechanischer Stimulation des geschädigten Innenohres durch Vibrationsanregung des Mittelohres,
- Fig. 8: eine Ausführungsform eines vollimplantierbaren Hörsystems mit mehrkanaliger elektrischer Reizung des Innenohres über ein intracochleäres Elektrodenarray,
- Fig. 9: eine Ausführungsform eines vollimplantierbaren Hörsystems mit kombinierter mehrkanaliger elektrischer und einkanaliger elektromechanischer Reizung des Innenohres über ein iritracochleäres Elektrodenarray in Verbindung mit einer mechanischen Anregung des Mittelohres,
- Fig. 10: eine Ausführungsform eines vollimplantierbaren Hörsystems mit mehrkanaliger elektromechanischer Reizung des Innenohres über ein intracochleäres Wandlerarray,
- Fig. 11: eine Ausführungsform eines vollimplantierbaren Hörsystems mit direkter mehrkanaliger elektromechanischer und elektrischer Reizung des Innenohres und
- Fig. 12: eine schematische Darstellung eines im Kopf eines Trägers implantierten binauralen Hörsystems.

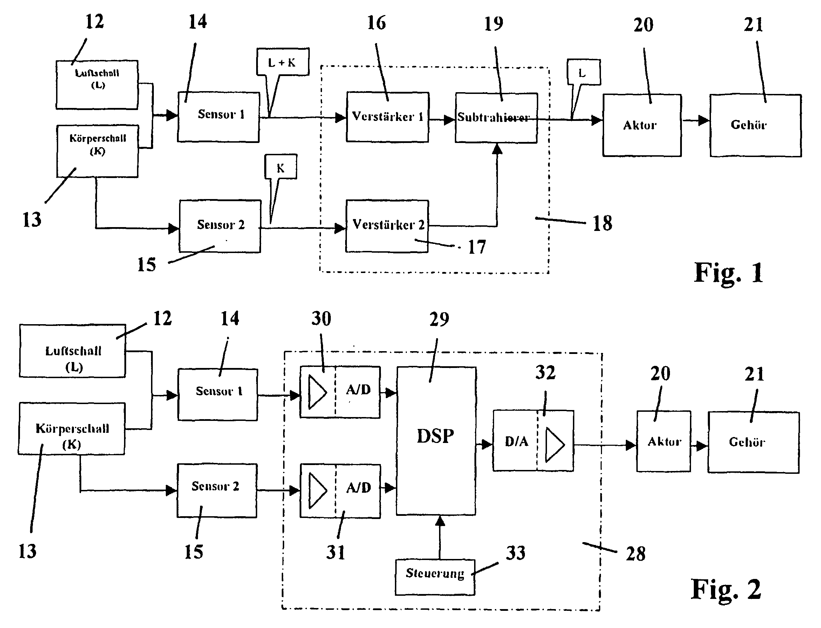

In dem Blockschaltbild eines voll implantierbaren Hörsystems gemäß Fig. 1 sind extern einfallender Luftschall (L) und im implantierten Zustand intern im Implantatträger auftretender Körperschall (K) mit den Blöcken 12 beziehungsweise 13 angedeutet. Das Hörsystem weist ein Mikrofon in Form eines Sensors 14 auf, der sowohl Luftschall als auch Körperschall (in Fig. 1 mit "L + K" bezeichnet) aufnimmt. Das Hörsystem ist mit einem weiteren Sensor 15 ausgestattet, der so ausgebildet und angeordnet ist, dass er im implantierten Zustand im wesentlichen nur internen Körperschall (K) aufnimmt. Die Sensoren 14, 15 wandeln den einfallenden Schall in entsprechende elektrische Signale um. Jedem der Sensoren 14, 15 ist ein Verstärker 16 beziehungsweise 17 in einem insgesamt mit 18 bezeichneten Elektronikmodul nachgeschaltet. Die Verstärker 16, 17 sorgen für eine Verstärkung des betreffenden elektrischen Sensorsignals von dem Sensor 14 beziehungsweise 15. Die Ausgangssignale der Verstärker 16, 17 gehen einem Subtrahierer 19 zu, der die Differenz der beiden gewichteten Sensorsignale erzeugt, wobei für eine zweckentsprechende Gewichtung der elektrischen Sensorsignale gesorgt wird. Im Extremfall kann so bewirkt werden, dass von dem Subtrahierer 19 nur das Luftschallsignal L an eine implantatspezifische Aktorik 20 und damit an das geschädigte Gehör 21 weitergegeben wird.

Der Subtrahierer 19 kann, wie oben beschrieben, spektral gewichtet Amplituden- und Phaseninformationen berücksichtigen, so dass durch Einstellung dieser Parameter (nicht dargestellt) eine beliebige, individuell einstellbare Mischung der Signale L und K an das Gehör weitergegeben werden kann.

Fig. 2 zeigt eine bevorzugte Ausführungsform eines voll implantierbaren Hörsystems, bei der ein Elektronikmodul 28 mit einem digitalen Signalprozessor (DSP) 29 vorgesehen ist. Zu dem Elektronikmodul 28 gehören ferner zwischen die Ausgänge der Sensoren 14, 15 und Eingänge des Signalprozessors 29 geschaltete A/D-Einheiten 30 und 31 sowie eine zwischen einem Ausgang des Signalprozessors 29 und dem Eingang der Aktorik 20 liegende D/A-Einheit 32. Die A/D-Einheiten 30, 31 sorgen für eine Verstärkung und Analog-Digital-Wandlung der analogen Sensorsignale. In dem digitalen Signalprozessor 29 werden die digital gewandelten Sensorsignale wie angegeben entsprechend bearbeitet, wobei der Signalprozessor 29 auch die audiologische Signalverarbeitung des jeweiligen Implantatsystems beinhaltet. Das beziehungsweise die digitale(n) Ausgangssignal(e) des Signalprozessors 29 wird (werden) in der D/A-Einheit 32 analog rückgewandelt und über einen an die jeweilige Stimulationsform des Implantatsystems angepassten Treiber verstärkt an die in Fig. 2 nur einkanalig dargestellte ausgangsseitige Aktorik 20 geleitet. Dem Signalprozessor 29 ist eine insgesamt mit 33 bezeichnete Steuerung zugeordnet.

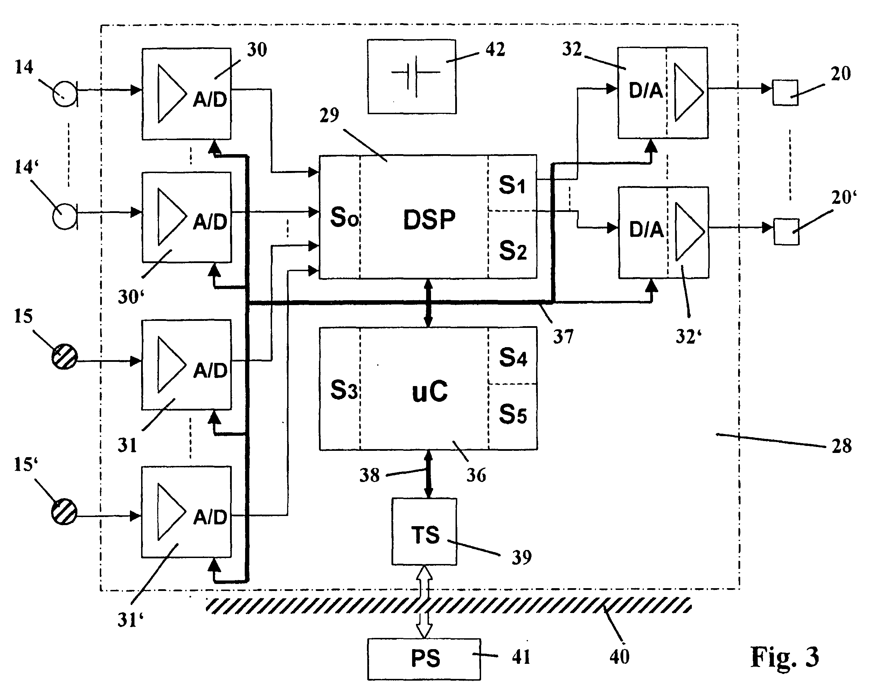

In Fig. 3 sind Einzelheiten eines bevorzugten Aufbaus des signalverarbeitenden Elektronikmoduls 28 entsprechend Fig. 2 dargestellt. Ein oder mehrere Mikrofone (Sensoren) 14, 14' nehmen das externe Luftschall- und interne Körperschallsignale auf und wandeln sie in entsprechende elektrische Signale um. Diese Sensorsignale werden in A/D-Einheiten 30, 30' vorverstärkt und analog-digital (A/D) gewandelt. Ein oder mehrere weitere Sensoren 15, 15' nehmen den internen Körperschall auf. Diese Sensorsignale werden in A/D-Einheiten 31, 31' ebenfalls vorverstärkt und analog-digital gewandelt. Alle digitalen Sensorsignale werden in dem digitalen Signalprozessor (DSP) 29 weiterverarbeitet.

Der Signalprozessor 29 enthält einen nicht überschreibbaren Festspeicherbereich S₀, in welchem die für einen "Minimalbetrieb" des Systems erforderlichen Anweisungen und Parameter gespeichert sind, sowie mindestens einen wiederholt beschreibbaren Speicherbereich S₁, in dem die Betriebssoftware der bestimmungsgemäßen Funktion beziehungsweise Funktionen des Implantatsystems abgelegt sind. Vorzugsweise ist dieser Speicherbereich doppelt vorhanden (S₁ und S₂). Der wiederholt beschreibbare Programmspeicher zur Aufnahme der Betriebssoftware kann auf EEPROM-Basis oder RAM-Zellen basieren, wobei in diesem Fall dafür gesorgt sollte, dass dieser RAM-Bereich immer durch das Energieversorgungssystem "gepuffert" ist. In den Speichern S₁ und/oder S₂ können auch von außen veränderliche, patientenspezifische Daten, wie zum Beispiel audiologische Anpaßparameter, abgelegt sein.

Die digitalen Ausgangssignale des Signalprozessors 29 werden in den D/A-Einheiten 32, 32' in Analogsignale umgewandelt und auf den zur Ansteuerung der Aktoren 20, 20' gewünschten Pegel gebracht. Die aktorische Ausgangsseite des Implantatsystems kann von einem oder mehreren elektromechanischen oder elektroakustischen Wandlern oder von elektrischen Reizelektroden oder von beliebige Kombinationen der genannten Ausgangsstimulatoren gebildet sein. Die Einheiten 32, 32' können unter Umständen entfallen, wenn zum Beispiel bei Verwendung eines elektromagnetischen intracochleären Ausgangswandlers ein beispielsweise pulsweitenmoduliertes, serielles digitales Ausgangssignal des Signalprozessors 29 direkt an den oder die Aktoren 20, 20' übermittelt wird.

Damit die software-basierten Algorithmen zur möglichst optimalen Stimulation des geschädigten Gehörs auch postoperativ implementiert werden können, enthält das System nach Fig. 3 einen weiteren Mikroprozessorbaustein, zum Beispiel einen Mikrokontroller (µC) 36. Der Mikrocontroller 36 steuert die A/D-Konverter der Sensor-Vorverarbeitungs-Einheiten 30, 30', 31, 31', die D/A-Konverter der Einheiten 32, 32' zur Ansteuerung der Aktoren 20, 20' und den Signalprozessor 29 selbst über einen bidirektionalen Datenbus 37an, und er ist dafür mit einem oder zwei zugehörigen Speichern S₄ beziehungsweise S₅ ausgestattet. In dem beziehungsweise den Speicherbereichen S₄ und S₅ können insbesondere die Betriebsoftwareanteile des Implantatmanagementsystems abgelegt sein, zum Beispiel Verwaltungs-, Überwachungs- und Telemetriefunktionen. Ferner weist der Mikrocontroller 36 einen wiederholt beschreibbaren Speicher S₃ auf, in welchem ein Arbeitsprogramm für den Mikrocontroller 36 abgelegt ist.

Der Mikrocontroller 36 kommuniziert über einen Datenbus 38 mit einem Telemetriesystem (TS) 39. Dieses Telemetriesystem 39 kommuniziert seinerseits durch die bei 40 angedeutete geschlossene Haut beispielweise über eine nicht dargestellte induktive Spulenkopplung drahtlos bidirektional mit einem externen Programmiersystem (PS) 41. Das Programmiersystem 41 kann vorteilhaft ein PC-basiertes System mit entsprechender Programmier-, Bearbeitungs-, Darstellungs- und Verwaltungssoftware sein. Über diese Telemetrieschnittstelle wird im Bedarfsfall die zu verändernde beziehungsweise ganz auszutauschende Betriebssoftware des Implantatsystems übertragen und zunächst in dem Speicherbereich S₄ und/oder S₅ des Mikrocontrollers 36 zwischengespeichert. So kann zum Beispiel der Speicherbereich S₅ für eine komplementäre Ablage der von dem externen System übermittelten Daten benutzt werden, und eine einfache Verifikation der Softwareübertragung durch einen Lesevorgang über die Telemetrieschnittstelle kann durchgefuhrt werden, um die Koinzidenz der Inhalte der Speicherbereiche S₄ und S₅ zu uberprufen, bevor der Inhalt des wiederholt beschreibbaren Speicher S₃ geändert oder ausgetauscht wird.

Die Betriebssoftware des implantierbaren Hörsystems soll gemäß der vorliegend verwendeten Nomenklatur sowohl die Betriebssoftware des Mikrocontrollers 36 (zum Beispiel Housekcepiftg-Funktionen, wie Energiemanagement oder Telemetriefunktionen) als auch die Betriebssoftware des digitalen Signalprozessors 29 umfassen. So kann zum Beispiel eine einfache Verifikation der Softwareübertragung durch einen Lesevorgang über die Telemetrieschnittstelle durchgeführt werden, bevor die Betriebssoftware oder die entsprechenden Signalverarbeitungsanteile dieser Software über den Datenbus 37 in den Programmspeicherbereich S₁ des digitalen Signalprozessors 29 übertragen werden. Ferner kann auch das Arbeitsprogramm für den Mikrocontroller 36, das beispielsweise in dem wiederholt beschreibbaren Speicher S₃ eingespeichert ist, über die Telemetrieschnittstelle 39 mit Hilfe der externen Einheit 41 ganz oder teilweise geändert oder ausgetauscht werden.

Alle elektronischen Komponenten des Implantatsystems werden durch eine primäre oder sekundäre Batterie 42 mit elektrischer Betriebsenergie versorgt.

Die beschriebene Lösung erlaubt eine Anpassung des Hörsystems an Gegebenheiten, die erst nach der Implantation erfassbar sind. So sind beispielsweise bei einem implantierbaren Hörsystem zur Rehabilitation einer monauralen oder binauralen Innenohrstörung sowie eines Tinnitus mit mechanischer Stimulation und/oder elektrischer Reizung des Innenohres die sensorischen (Luft- und Körperschallsensoren) und aktorischen (Ausgangsstimulator) biologischen Schnittstellen immer abhängig von den anatomischen, biologischen und neurophysiologischen Gegebenheiten, zum Beispiel von dem interindividuellen Einheilprozeß. Diese Schnittstellenparameter können individuell insbesondere auch zeitvariant sein. So können beispielsweise das Übertragungsverhalten eines implantierten Luft- oder Körperschallsensors aufgrund von Gewebebelägen und das Übertragungsverhalten eines an das Innenohr angekoppelten elektromechanischen Wandlers aufgrund unterschiedlicher Ankopplungsqualität interindividuell und individuell variieren. Solche Unterschiede der Schnittstellenparameter, die sich normalerweise nicht einmal durch den Austausch des Implantats mindern beziehungsweise eliminieren ließen, können vorliegend durch Veränderung beziehungsweise Verbesserung der Signalverarbeitung des Implantats optimiert werden.

Bei einem implantierbaren Hörsystem kann es auch sinnvoll oder notwendig werden, nach Implantation verbesserte Signalverarbeitungsalgorithmen zu implementieren. Dabei sind insbesondere zu nennen:
- Verfahren zum Optimieren des Verhältnisses von Luft- und Körperschallsignalen,
- Sprachanalyseverfahren (zum Beispiel Optimierung einer Fast-Fourier-Transformation (FFT)),
- statische oder adaptive Störschallerkennungsverfahren,
- statische oder adaptive Störschallunterdrückungsverfahren,
- Verfahren zur Optimierung des systeminternen Signal-Rauschabstandes,
- optimierte Signalverarbeitungsstrategien bei progredienter Hörstörung,
- ausgangspegelbegrenzende Verfahren zum Schutz des Patienten bei Implantatfehlfunktionen bzw. externen Fehlprogrammierungen,
- Verfahren zur Vorverarbeitung mehrerer Luft- und/oder Körperschallsignale, insbesondere bei binauraler Positionierung der Sensoren,
- Verfahren zur binauralen Verarbeitung zweier oder mehrerer Luftschall-Sensorsignale bei binauraler Sensorpositionierung, zum Beispiel Optimierung des räumlichen Hörens beziehungsweise der Raumorientierung,
- Phasen- beziehungsweise Gruppenlaufzeit-Optimierung bei binauraler Signalverarbeitung,
- Verfahren zur optimierten Ansteuerung der Ausgangsstimulatoren, insbesondere bei binauraler Positionierung der Stimulatoren

Mit dem vorliegenden System lassen sich auch nach der Implantation unter anderem die folgenden Signalverarbeitungsalgorithmen implementieren:
- Verfahren zur Rückkopplungsunterdrückung beziehungsweise -minderung,
- Verfahren zur Optimierung des Betriebsverhaltens des bzw. der Ausgangswandler (zum Beispiel Frequenz- und Phasengangoptimierung, Verbesserung des Impulsübertragungsverhaltens),
- Sprachsignal-Kompressionsverfahren bei Innenohrschwerhörigkeiten,
- Signalverarbeitungsmethoden zur Recruitment-Kompensation bei Innenohrschwerhörigkeiten.

Des weiteren ist bei Implantatsystemen mit einer sekundären Energieversorgungseinheit, das heißt einem nachladbaren Akkumulatorsystem, aber auch bei Systemen mit primärer Batterieversorgung zu erwarten, dass die elektrischen Energiespeicher mit voranschreitender Technologie immer größere Lebensdauern und damit steigende Verweilzeiten im Patienten ermöglichen. Es ist ferner davon auszugehen, dass die Grundlagen und Applikationsforschung für Signalverarbeitungsalgorithmen schnelle Fortschritte macht. Die Notwendigkeit oder der Patientenwunsch einer Betriebssoftwareanpassung beziehungsweise -veränderung wird daher voraussichtlich vor Ablauf der Lebensdauer der implantatinternen Energiequelle eintreten. Das vorliegend beschriebene System erlaubt eine derartige Anpassung der Betriebsprogramme des Implantats auch im bereits implantierten Zustand.

Die entsprechenden signalverarbeitenden Softwaremodule des digitalen Signalprozessors 29 können statisch und dynamisch ausgelegt sein. Unter "statisch" soll vorliegend verstanden werden, dass die Softwaremodule aufgrund wissenschaftlicher Erkenntnisse einmalig im Programmspeicher des Signalprozessors 29 abgelegt werden und unverändert bleiben. Unter "dynamisch" soll dagegen vorliegend verstanden werden, dass diese Softwaremodule "lernfähig" sind, um der jeweils gewünschten Funktion zeitlich iterativ möglichst optimal nahe zu kommen. Dies bedeutet, dass die Softwaremodule adaptiv ausgelegt sein können und die Parameteranpassung durch "Training" durch den Implantatträger und gegebenenfalls weitere Hilfsmittel wie Rehabilitationsprogramme vorgenommen wird. Weiterhin kann ein Softwaremodul enthalten sein, welches das Ziel einer möglichst optimalen Hörversorgung auf der Basis eines lernfähigen neuronalen Netzwerkes approximiert. Das Training dieses neuronalen Netzes kann wieder durch den Implantatträger erfolgen und/oder unter Zuhilfenahme weiterer externer Hilfsmittel.

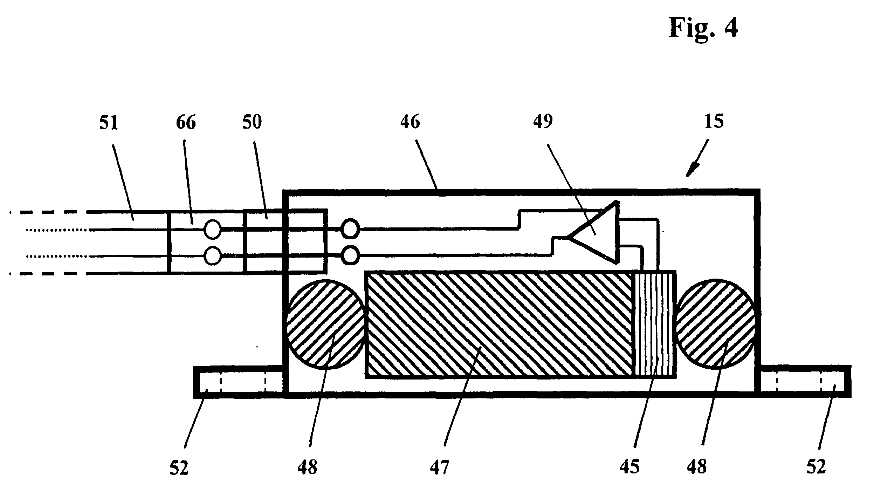

Fig. 4 zeigt beispielhaft und schematisch eine mögliche Ausführung eines separaten Körperschallsensors 15 mit einem piezoelektrischen Wandlerelement 45. In einem hermetisch dichten und biokompatiblen Gehäuse 46 ist eine träge Masse 47 mit dem Piezoelement 45 mechanisch verbunden. Dieser Verbund ist über elastische Elemente 48 in dem Gehäuse 46 eingebaut. Mechanische Schwingungen, wie zum Beispiel Körperschallschwingungen, die auf das Gehäuse 46 einwirken, führen zu dynamischen Relativbewegungen der trägen Masse 47 und somit zu Auslenkungen des Piezoelementes 45. Die dabei entstehenden Spannungssignale des Piezoelementes 45 werden über eine in dem Gehäuse 46 integrierte Impedanzwandler- und Vorverstärker-Einheit 49 abgenommen. Das elektrische Sensorausgangssignal wird über hermetisch dichte Signaldurchführungen 50 und eine Implantatsensorleitung 51 zu dem Elektronikmodul 18 beziehungsweise 28 des entsprechenden Implantatsystems geführt. Die energetische Versorgung der Impedanzwandler- und Vorverstärker-Einheit 49 kann bei einer zweipoligen Sensorleitung 51 über eine Phantomspeisung erfolgen. Zum Befestigen, beispielsweise Anschrauben, des Körperschallsensors 15 ist das Gehäuse 46 mit einem abstehenden Flansch 52 versehen.

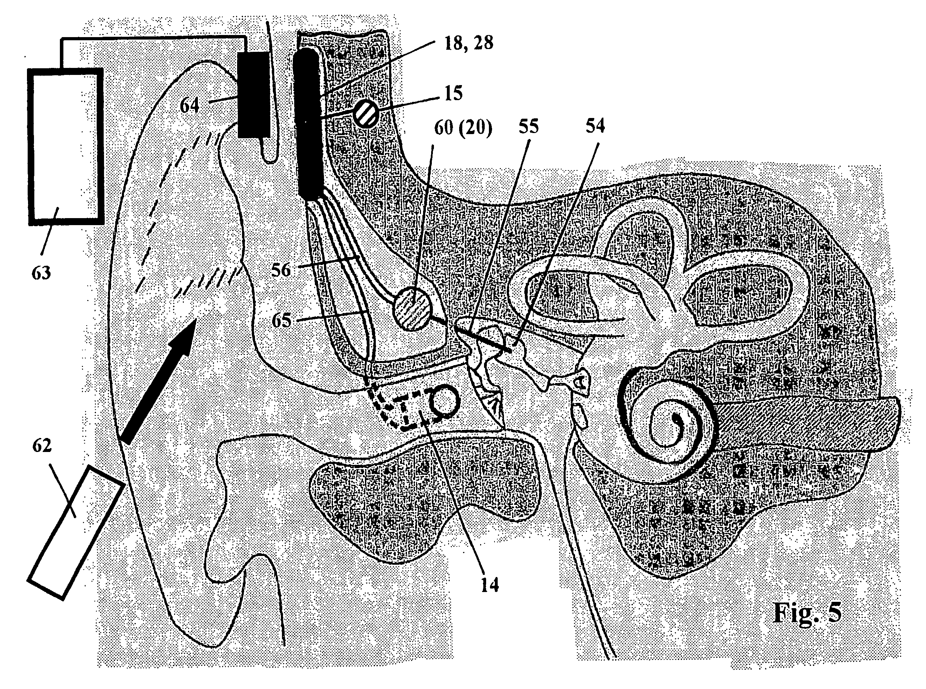

Fig. 5 zeigt schematisch den Aufbau eines vollständig implantierbaren Hörsystems mit einer einkanaligen elektromechanischen Stimulation des geschädigten Innenohres durch Vibrationsanregung des Mittelohres, wobei als ausgangsseitiger Aktor (20 in den Figuren 1 bis 3) ein beispielsweise an den Amboss 54 über eine Koppelstange 55 angekoppelter elektromechanischer Wandler 60 vorgesehen ist. Bei dem Wandler 60 kann es sich generell um einen beliebigen elektromagnetischen, elektrodynamischen, piezoelektrischen, magnetostriktiven oder dielektrischen (kapazitiven) Wandler handeln. Geeignet ist insbesondere ein piezoelektrisches Wandlersystem der aus US-A-5-277-694 bekannten Art. Ein solcher bevorzugter Wandler weist ein mit Inertgas gefülltes biokompatibles, zylindrisches Gehäuse aus elektrisch leitendem Material, beispielsweise Titan, auf, in dem eine schwingungsfähige, elektrisch leitende, vorzugsweise kreisrunde Membran an ihrem Außenrand fest eingespannt ist. Mit der einen Seite der Membran steht eine dünne Scheibe aus piezoelektrischem Material, zum Beispiel Blei-Zirkonat-Titanat, in elektrisch leitender Verbindung. Auf der von der Membran abgewendeten Seite ist die Piezoscheibe mit einem dünnen, flexiblen Draht kontaktiert, der über eine hermetische Gehäusedurchführung mit einer Wandlerleitung 56 verbunden ist. Das Anlegen einer elektrischen Spannung an den Wandler 60 bewirkt ein Durchbiegen des Hetero-Verbundes aus Membran und Piezoscheibe. Ein ausgangsseitiger elektromechanischer Wandler 60 dieser Art hat typischerweise eine relativ hohe mechanische Ausgangsimpedanz, die vorzugsweise höher ist als die mechanische Lastimpedanz der im implantierten Zustand an den Wandler angekoppelten biologischen Mittel- und/oder Innenohrstruktur.

Der in Fig. 5 nur schematisch dargestellte Wandler 60 kann auch in der in US-A-6 123 660 erläuterten Weise dahingehend modifiziert sein, dass an der von der Membran abgewendeten Seite der Piezoscheibe ein Permanentmagnet angebracht ist, der nach Art eines elektromagnetischen Wandlers mit einer Elektromagnetspule zusammenwirkt. Ein solcher kombinierter piezoelektrischer/elektromagnetischer Wandler ist besonders im Hinblick auf ein breites Frequenzband und auf die Erzielung relativ großer Schwingungsamplituden mit verhältnismäßig kleiner zugeführter Energie von Vorteil. Ein vorliegend als Aktor 20 geeigneter elektromechanischer Wandler ist in US-A-6 162 169 beschrieben.

Zum Ankoppeln des elektromechanischen Wandlers 60 an das Mittel- oder Innenohr eignen sich besonders Koppelanordnungen gemäß US-A-5 941 814, bei denen ein Koppelelement außer einem Ankoppelteil für den betreffenden Ankoppelort eine Crimphülse aufweist, die zunächst lose auf einen mit rauher Oberfläche versehenen stabförmigen Teil der mit dem Wandler verbundenen Koppelstange 55 aufgeschoben ist. Beim Implantieren kann die Crimphülse gegenüber der Koppelstange einfach verschoben und gedreht werden, um das Ankoppelteil des Koppelelementes mit dem beabsichtigten Ankoppelort exakt auszurichten. Dann wird die Crimphülse fixiert, indem sie mittels eines Crimpwerkzeuges plastisch kaltverformt wird. Alternativ kann das Koppelelement mit Bezug auf die Koppelstange auch mittels einer zuziehbaren Bandschlaufe festgelegt werden.

Weitere vorliegend bevorzugt verwendbare Koppelanordnungen sind im einzelnen in DE-A-199 23 403, DE-A-199 35 029, DE-C-199 31 788, DE-A-199 48 336 und DE-A-199 48 375 beschrieben. So kann gemäß DE-A-199 23 403 ein Koppelelement an seinem Ankoppelende eine Kontaktfläche aufweisen, die eine an die Oberflächenform der Ankoppelstelle anpassbare oder angepasste Oberflächenform sowie eine solche Oberflächenbeschaffenheit und Oberflächengröße aufweist, dass es durch Anlegen des Ankoppelendes an die Ankoppelstelle zu einer dynamischen Zug-Druck-Kraftkopplung von Koppelelement und Ossikelkette durch Oberflächenadhäsion kommt, die für eine sichere gegenseitige Verbindung von Koppelelement und Ossikelkette ausreicht. Das Koppelelement kann mit einem im implantierten Zustand an der Ankoppelstelle anliegenden Dämpfungsglied mit entropieelastischen Eigenschaften versehen sein, um eine optimale Schwingungsform der Steigbügelfußplatte oder einer das runde Fenster oder ein artifizielles Fenster in der Cochlea, im Vestibulum oder im Labyrinth abschließenden Membran zu erreichen und das Risiko einer Beschädigung der natürlichen Strukturen im Bereich der Ankoppelstelle während und nach der Implantation besonders gering zu halten (DE-A-199 35 029).

Das Koppelelement kann entsprechend DE-C-199 31 788 mit einer Stellvorrichtung zum wahlweisen Verstellen des Koppelelements zwischen einer Offenstellung, in welcher das Koppelelement in und außer Eingriff mit der Ankoppelstelle bringbar ist, und einer Schließstellung versehen sein, in welcher das Koppelelement im implantierten Zustand mit der Ankoppelstelle in Kraft- und/oder Formschlussverbindung steht.

Zum mechanischen Ankoppeln des elektromechanischen Wandlers 60 an eine vorgewählte Ankoppelstelle an der Ossikelkette eignet sich ferner eine Koppelanordnung (DE-A-199 48 336), die zusätzlich zu der von dem Wandler in mechanische Schwingungen versetzbaren Koppelstange 55 ein mit der vorgewählten Ankoppelstelle in Verbindung bringbares Koppelelement aufweist, wobei die Koppelstange und das Koppelelement über wenigstens eine Kupplung miteinander verbunden sind und zumindest ein im implantierten Zustand an der Ankoppelstelle anliegender Abschnitt des Koppelelements zur verlustarmen Schwingungseinleitung in die Ankoppelstelle ausgelegt ist, wobei eine erste Kupplungshälfte der Kupplung eine Außenkontur mit mindestens näherungsweise der Gestalt einer Kugelkalotte aufweist, die in einer zur Außenkontur wenigstens teilweise komplementären Innenkontur einer zweiten Kupplungshälfte aufnehmbar ist, und wobei die Kupplung gegen Reibkräfte reversibel verschwenk- und/oder drehbar, jedoch bei im implantierten Zustand auftretenden dynamischen Kräften im Wesentlichen starr ist. Entsprechend einer abgewandelten Ausführungsform einer solchen Koppelanordnung (DE-A-199 48 375) hat eine erste Kupplungshälfte der Kupplung eine Außenkontur mit mindestens näherungsweise zylindrischer, vorzugsweise kreiszylindrischer, Gestalt, die in einer zur Außenkontur wenigstens teilweise komplementären Innenkontur einer zweiten Kupplungshälfte aufnehmbar ist, wobei ein im implantierten Zustand an der Ankoppelstelle anliegender Abschnitt des Koppelelements zur verlustarmen Schwingungseinleitung in die Ankoppelstelle ausgelegt ist, wobei im implantierten Zustand eine Übertragung von dynamischen Kräften zwischen den beiden Kupplungshälften der Kupplung im Wesentlichen in Richtung der Längsachse der ersten Kupplungshälfte erfolgt, und wobei die Kupplung reversibel an- und abkuppelbar sowie reversibel linear und/oder rotatorisch mit Bezug auf eine Längsachse der ersten Kupplungshälfte verstellbar, jedoch bei im implantierten Zustand auftretenden dynamischen Kräften starr ist.

Zu dem in Fig. 5 dargestellten vollständig implantierbaren Hörsystem gehören ferner ein subkutan in der hinteren Gehörgangswand implantierbarer Luftschallsensor (Mikrofon) 14 zur Luftschallaufnahme, ein separat von dem Elektronikmodul 18 oder 28 im knöchernen Bereich des Mastoids implantierbarer Körperschallsensor 15 beispielsweise der in Fig. 4 veranschaulichten Art, eine drahtlose Fernbedienung 62 zur Steuerung der Implantatfunktionen durch den Implantatträger, sowie - bei Vorhandensein einer implantatinternen sekundären Batterie 42 - ein transkutanes Ladesystem mit einem Ladegerät 63 und einer Ladespule 64 zum Nachladen der der Energieversorgung des Hörsystems dienenden Batterie 42 (Fig. 3).

Das Luftschallsensor 14 kann vorteilhaft in der aus EP-A-0 831 673 bekannten Weise aufgebaut und mit einer Mikrofonkapsel, die in einem Gehäuse allseitig hermetisch dicht untergebracht ist, sowie mit einer elektrischen Durchführungsanordnung zum Durchfuhren mindestens eines elektrischen Anschlusses von dem Innenraum des Gehäuses zu dessen Außenseite versehen sein, wobei das Gehäuse mindestens zwei Schenkel aufweist, die in einem Winkel mit Bezug aufeinander ausgerichtet sind, wobei der eine Schenkel die Mikrofonkapsel aufnimmt und mit einer Schalleintrittsmembran versehen ist, wobei der andere Schenkel die elektrische Durchführungsanordnung enthält und gegenüber der Ebene der Schalleintrittsmembran zurückversetzt ist, und wobei die Geometrie des Mikrofongehauses so gewählt ist, dass bei Implantation des Mikrofons in der Mastoidhöhle der die Schalleintrittsmembran enthaltende Schenkel vom Mastoid aus in eine artifizielle Bohrung in der hinteren, knöchernen Gehörgangswand hineinragt und die Schalleintrittsmembran die Haut der Gehörgangswand berührt. Zur Festlegung des implantierten Luftschallsensors 14 kann zweckmäßig ein Fixationselement der aus US-A-5 999 632 bekannten Art vorgesehen sein, das eine Manschette aufweist, die mit einem zylindrischen Gehäuseteil den die Schalleintrittsmembran enthaltenden Schenkel umschließt und mit gegen die der Gehörgangshaut zugewendete Seite der Gehörgangswand anlegbaren, vorspringenden, elastischen Flanschteile versehen ist. Dabei beinhaltet das Fixationselement vorzugsweise eine Halterung, welche die genannten Flanschteile vor der Implantation entgegen einer elastischen Rückstellkraft der Flanschteile in einer das Durchstecken durch die Bohrung der Gehörgangswand erlaubenden umgebogenen Stellung hält.

Die an den Ausgang des Ladegerätes 63 angeschlossene Ladespule 64 bildet vorzugsweise in der aus US-A-5 279 292 bekannten Art Teil eines Sende-Serienresonanzkreises, der mit einem nicht veranschaulichten Empfangs-Serienresonanzkreis induktiv gekoppelt werden kann. Der Empfangs-Serienresonanzkreis kann Teil des implantierbaren Elektronikmoduls 18 beziehungsweise 28 (Figuren 1 und 2) sein und entsprechend US-A-5 279 292 eine Konstantstromquelle für die Batterie 42 (Fig. 3) bilden. Dabei liegt der Empfangs-Serienresonanzkreis in einem Batterie-Ladestromkreis, der in Abhängigkeit von der jeweiligen Phase des in dem Ladestromkreis fließenden Ladestromes über den einen oder den anderen Zweig einer Vollweg-Gleichrichterbrücke geschlossen wird.

Das Elektronikmodul 18 beziehungsweise 28 ist bei der Anordnung nach Fig. 5 über eine Mikrofonleitung 65 an den Luftschallsensor 14 angeschlossen. Der Körperschallsensor 15 kann an das Elektronikmodul 18 beziehungsweise 28 über eine in Fig. 4 bei 66 angedeutete lösbare Steckverbindung angeschlossen sein.

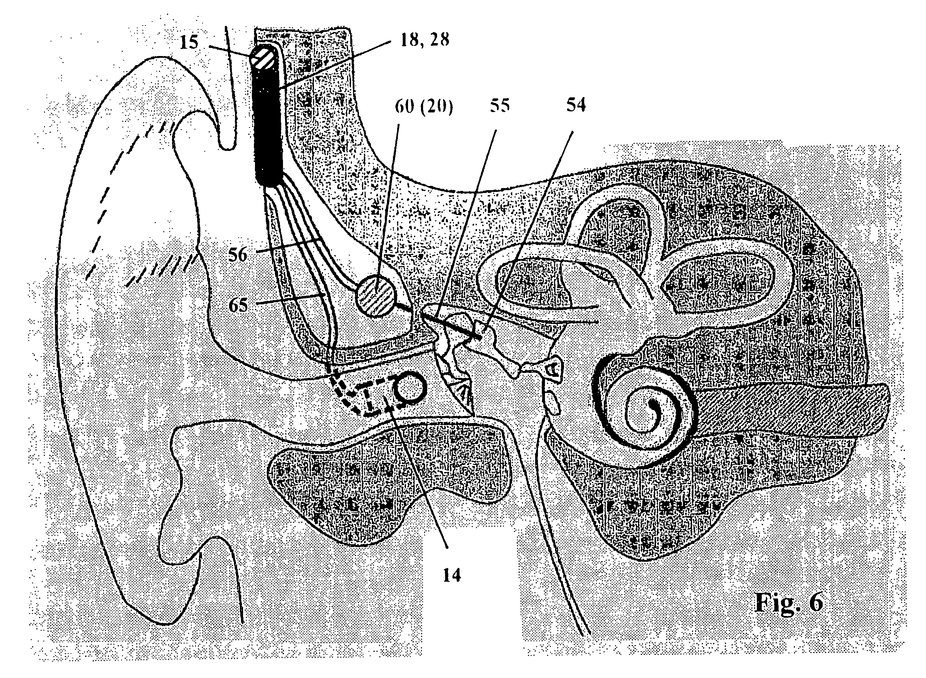

Das Hörsystem gemäß Fig. 6 unterscheidet sich von dem der Fig. 5 dadurch, dass der Körperschallsensor 15 in das Elektronikmodul 18 beziehungsweise 28 integriert ist und Körperschallschwingungen über die mechanische Kopplung des Elektronikmodulgehäuses zum Schädel empfängt. Hierfür ist der beispielsweise entsprechend Fig. 4 ausgebildete interne Körperschallsensor 15 in mechanischer Kopplung zu dem Gehäuse des Elektronikmoduls vorzusehen.

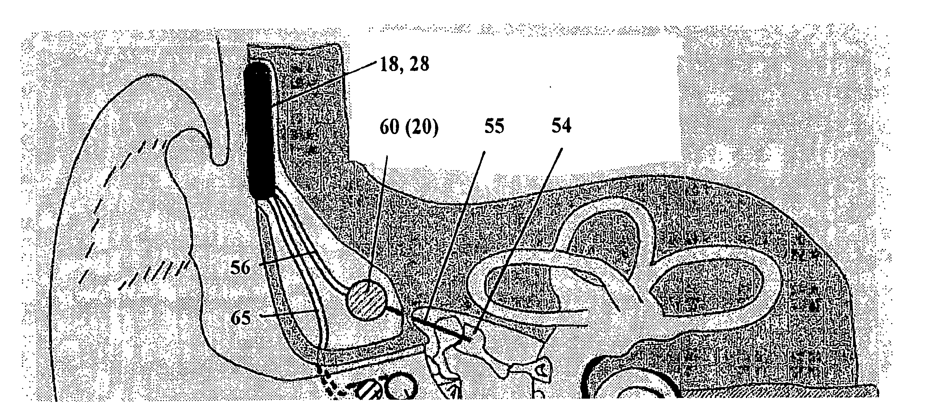

Bei dem in Fig. 7 veranschaulichten Hörsystem ist abweichend von den Anordnungen gemäß den Figuren 5 und 6 der Körperschallsensor 15 in das Gehäuse des in der hinteren Gehörgangswand implantierbaren Luftschallsensors 14 integriert. Hierfür ist der Körperschallsensor 15 in mechanischer Kopplung zu dem Mikrofongehäuse vorzusehen. Der Körperschallsensor 15 kann auch bei diesem Beispiel entsprechend Fig. 4 ausgebildet sein. Er wird über die Mikrofonleitung 65 angeschlossen. Bei gemeinsamen Massepotential beider Sensoren 14, 15 und energetischer Phantomspeisung ist hierbei nur eine dritte elektrische Ader in der Leitung 65 notwendig.

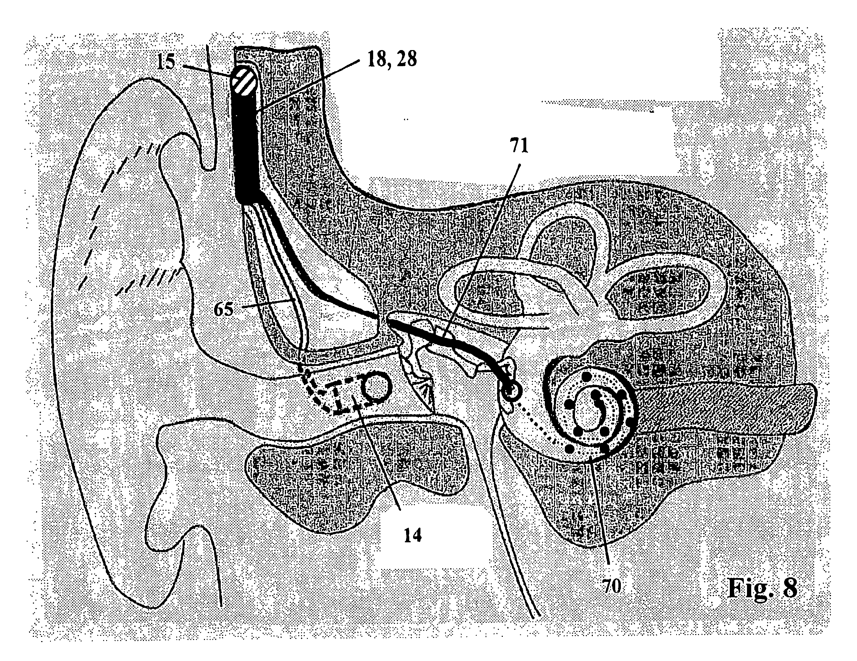

Das Hörsystem gemäß Fig. 8 ist als implantierbares Cochlea-Implantat ausgebildet. Es unterscheidet sich von dem Hörsystem der Fig. 6 dadurch, dass entsprechend US-A-5 814 095 als ausgangsseitiger Aktor (20 in den Figuren 1 bis 3) ein über eine Leitung 71 an das Elektronikmodul 18 beziehungsweise 28 angeschlossenes intracochleäres Elektrodenarray 70 für eine mehrkanalige elektrische Reizung des Innenohres vorgesehen ist.

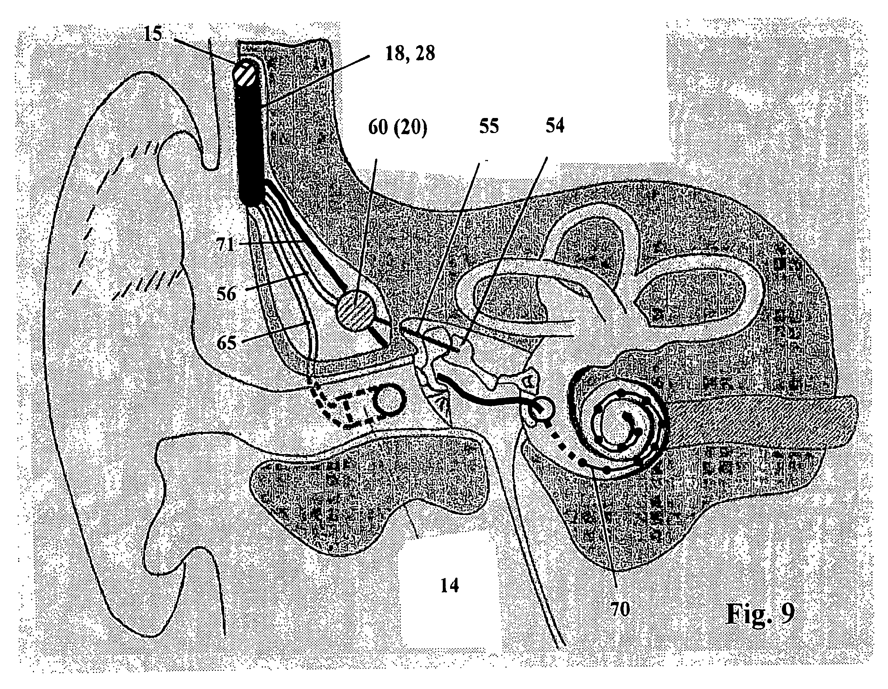

Das in Fig. 9 dargestellte Hörsystem weist als ausgangsseitige Aktoren das für eine mehrkanalige elektrische Reizung des Innenohres sorgende intracochleäre Elektrodenarray 70 der Fig. 8 in Kombination mit dem eine einkanalige elektromechanische Stimulation des geschädigten Innenohres bewirkenden elektromechanischen Wandler 60 der Figuren 5 bis 7 auf. Eine solche kombinierte Stimulation ist in EP-A-1 145 734 näher erläutert.

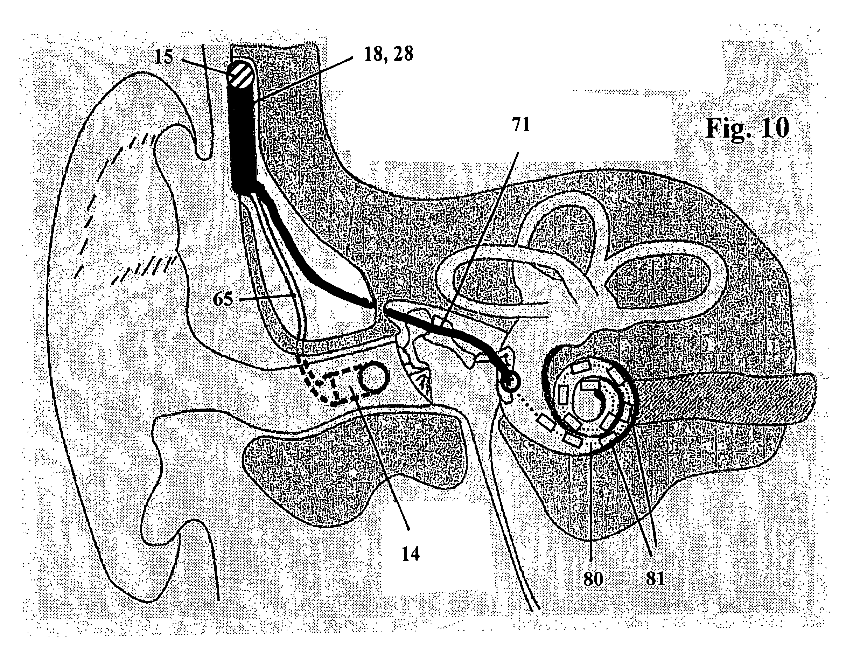

Das Hörsystem gemäß Fig. 10 unterscheidet sich von dem der Fig. 6 dadurch, dass als ausgangsseitiger Aktor (20 in den Figuren 1 bis 3) ein extra- oder intracochleäres Wandlerarray 80 mit mindestens zwei voneinander unabhängigen und örtlich getrennt angeordneten elektromechanischen Wandlern 81 vorgesehen ist, die in der Cochlea eine mehrkanalige mechanische Reizung bewirken. Dabei erfolgt mittels des Elektronikmoduls 18 beziehungsweise 28 die Ansteuerung der Wandler 81 des Wandlerarrays 80 vorzugsweise so, dass örtlich begrenzte Bereiche der Cochlea unter Ausbildung von Wanderwellen auf der Basilarmembran mechanisch stimuliert werden und dabei näherungsweise ein "gesunder" natürlicher cochleärer Verstärker simuliert wird, wie dies in EP-A-1 146 774 beschrieben ist.

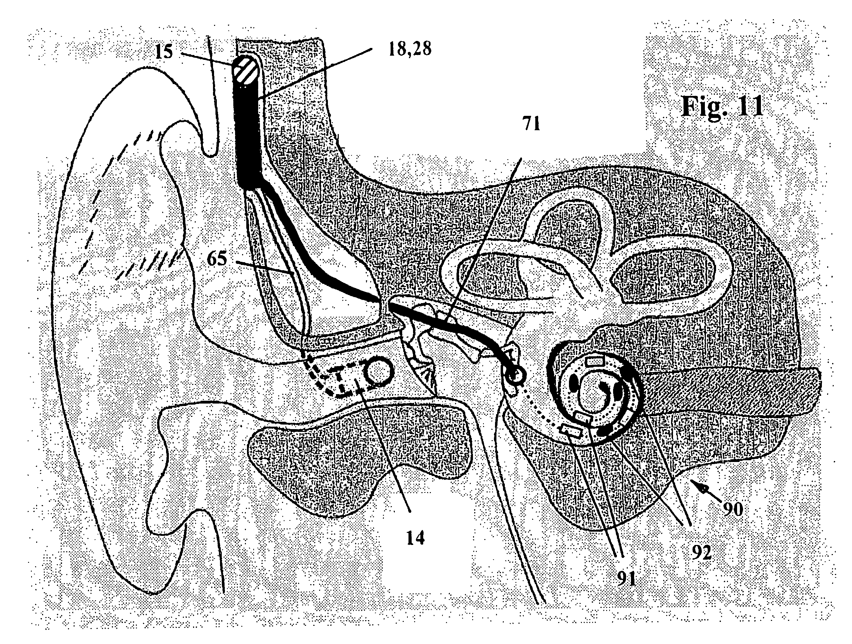

Bei dem in Fig. 11 veranschaulichten Hörsystem ist als ausgangsseitiger Aktor (20 in den Figuren 1 bis 3) ein intracochleäres Array 90 mit einer Folge von elektromechanischen Wandlern 91 und Reizelektroden 92 zur mehrkanaligen kombinierten elektromechanischen und elektrischen Reizung des Innenohres vorgesehen. Einzelheiten einer solchen aktorischen Anordnung sind in EP-A-1 145 733 erläutert.

Es versteht sich, dass bei den Hörsystemen der Figuren 8 bis 11 der Körperschallsensor 15 auch in der anhand der Figuren 5 und 7 beschriebenen Weise separat angeordnet (Fig. 5) oder zusammen mit dem Luftschallsensor 14 in einem gemeinsamen Gehäuse untergebracht (Fig. 7) werden kann. Wird bei dem Hörsystem der Fig. 5 das Elektronikmodul 18 beziehungsweise 28 aus einer primären Batterie gespeist, entfällt das Ladesystem mit Ladegerät 63 und Ladespule 64. Umgekehrt kann ein solches Ladesystem auch bei den Hörsystemen der Figuren 6 bis 11 vorgesehen sein, wenn dort die Energieversorgung des Implantats mittels einer sekundären Batterie erfolgt. Des weiteren versteht es sich, dass für eine Steuerung mittels der nur in Fig. 5 gezeigten drahtlosen Fernbedienung 62 auch im Falle der Hörsysteme der Figuren 6 bis 11 gesorgt sein kann.

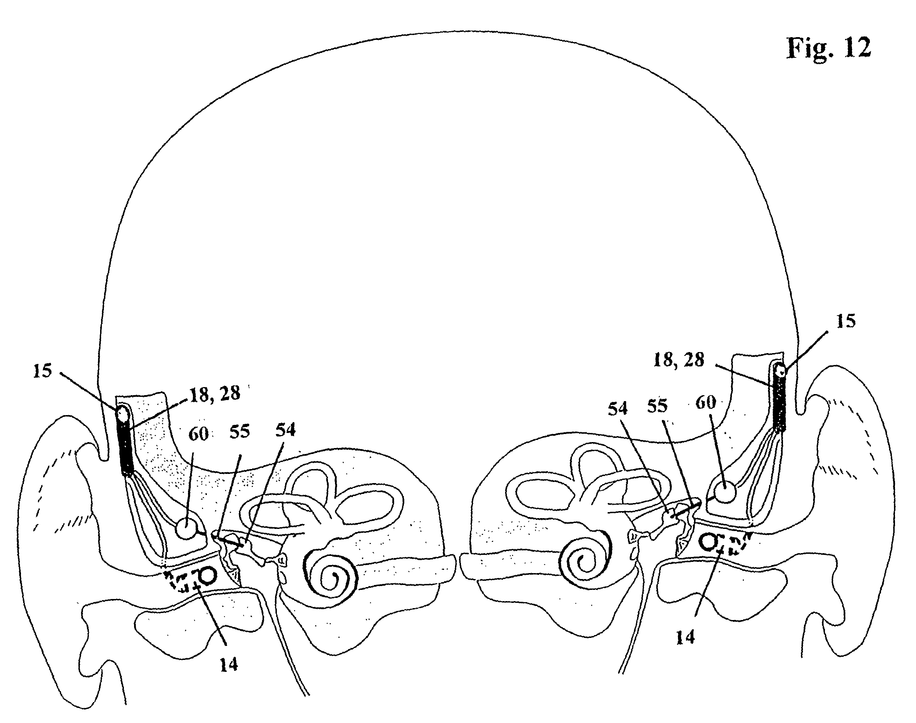

Alle vorliegend beschriebenen Hörsysteme können ferner als binaurales System zur Rehabilitation einer Hörstörung beider Ohren ausgelegt und dementsprechend mit zwei Systemeinheiten versehen sein, die jeweils einem der beiden Ohren zugeordnet sind. Ein solches binaurales Hörsystem ist in Fig. 12 dargestellt Dabei können, wie zum Beispiel in der vorstehend genannten EP-A-1 146 774 näher erläutert ist, die beiden Systemeinheiten einander im wesentlichen gleich sein. Es kann aber auch die eine Systemeinheit als Master-Einheit und die andere Systemeinheit als von der Master-Einheit gesteuerte Slave-Einheit ausgelegt sein. Die Signalverarbeitungsmodule der beiden Systemeinheiten können auf beliebige Weise, insbesondere über eine drahtgebundene implantierbare Leitungsverbindung oder über eine drahtlose Verbindung, vorzugsweise eine bidirektionale Hochfrequenzstrecke, eine körperschallgekoppelte Ultraschallstrecke oder eine die elektrische Leitfähigkeit des Gewebes des Implantatträgers ausnutzende Datenübertragungsstrecke, so miteinander kommunizieren, dass in beiden Systemeinheiten eine optimierte binaurale Signalverarbeitung und Ansteuerung der Aktoren 20, 20' (im Ausführungsbeispiel der Fig. 12 in Form der elektromechanischen Wandler 60) erreicht wird.

## Patentansprüche

1. Vollständig implantierbares Hörsystem zur Rehabilitation von Hörstörungen mit mindestens einem Sensor (14, 14'), der so ausgelegt ist, dasser mindestens Luftschall aufuirnmt und in elektrische Signale umwandelt, einem Elektronikmodul (18, 28) mit einer elektronischen Anordnung zur Audiosignalverarbeitung und -verstärkung, mit einer ausgangsseitigen aktorischen Anordnung (20, 20') zum Stimulieren des Mittel- oder Innenohres sowie mit einer elektrischen Energieversorgungseinheit (42), **dadurch gekennzeichnet, dass** das Hörsystem mindestens einen weiteren implantierbaren Sensor (15, 15') aufweist, der so ausgelegt ist, dasser mindestens körperschallinduzierte Signale aufnimmt und in elektrische Signale umwandelt, und dass durch elektronisches Verrechnen der Luftschallsensorsignale und der Körperschallsensorsignale in der elektronischen Anordnung das Verhältnis von Luft- und Körperschallsignalen individuell einstellbar ist.

2. Hörsystem nach Anspruch 1, **dadurch gekennzeichnet, dass** der mindestens eine Körperschallsensor (15, 15') nach dem elektromagnetischen, elektrodynamischen, piezoelektrischen, magnetostriktiven oder dielektrischen (kapazitiven) Wandlerprinzip arbeitet.

3. Hörsystem nach Anspruch 2, **dadurch gekennzeichnet, dass** der mindestens eine Körperschallsensor (15, 15') als On-Chip-Halbleiterwandler ausgebildet ist.

4. Hörsystem nach einem der Ansprüche 1 bis 3, **dadurch gekennzeichnet, dass** der Körperschallsensor (15, 15') einen mechano-elektrischen Wandler (45) aufweist, der an eine in einem Sensorgehäuse (46) schwingfähig aufgehängte träge Masse (47) angekoppelt ist

5. Hörsystem nach einem der vorhergehenden Ansprüche, **dadurch gekennzeichnet, dass** der Übertragungsbereich des mindestens einen Körperschallsensors (15, 15') zwischen 100 Hz bis 10 kHz liegt.

6. Hörsystem nach einem der vorhergehenden Ansprüche, **dadurch gekennzeichnet, dass** der mindestens eine Körperschallsensor (15, 15') so abgestimmt ist, dass seine erste mechanische Resonanzfrequenz am oberen spektralen Ende des Übertragungsfrequenzbereiches liegt.

7. Hörsystem nach einem der vorhergehenden Ansprüche, **dadurch gekennzeichnet, dass** der mindestens eine Körperschallsensor (15, 15') hermetisch dicht aufgebaut ist.

8. Hörsystem nach einem der vorhergehenden Ansprüche, **dadurch gekennzeichnet, dass** der mindestens eine Körperschallsensor (15, 15') separat von dem Eleklronikmodul (18, 28) implantierbar und vorzugsweise an das Eleh-tronikmodul (18, 28) mittels einer lösbaren Steckverbindung (66) angeschlossen ist.

9. Hörsystem nach einem der Ansprüche 1 bis 7, **dadurch gekennzeichnet, dass** der Körperschallsensor (15, 15') in das Elektronikmodul (18, 28) des Implantatsystems integriert ist.

10. Hörsystem nach einem der vorhergehenden Ansprüche, **dadurch gekennzeichnet, dass** das Verhältnis von Luft- und Körperschallsignalen über eine drahtlose Telemetrieeinrichtung einstellbar ist, mittels deren Daten zwischen einer externen Einheit und dem Implantat übertragbar sind.

11. Hörsystem nach einem der vorhergehenden Ansprüche, **dadurch gekennzeichnet, dass** es als binaurales System zur Rehabilitation einer Hörstörung beider Ohren ausgelegt ist, das zwei Systemeinheiten aufweist, die jeweils einem der beiden Ohren zugeordnet sind, wobei mindestens eine der beiden Systemeinheiten mit einem Körperschallsensor (15, 15') versehen ist.

12. Hörsystem nach einem der vorhergehenden Ansprüche, **dadurch gekennzeichnet, dass** der mindestens eine Luftschallsensor (14, 14') so ausgelegt und implantierbar ist, dass er eine Mischung von Luft- und Körperschall aufnimmt und in entsprechende elektrische Signale umwandelt.

13. Hörsystem nach einem der vorhergehenden Ansprüche, **dadurch gekennzeichnet, dass** der mindestens eine Körperschallsensor (15, 15') so ausgelegt und implantierbar ist, dass er im wesentlichen nur Körperschallsignale aufnimmt und in entsprechende elektrische Signale umwandelt.

14. Hörsystem nach einem der vorhergehenden Ansprüche, **dadurch gekennzeichnet, dass** die Amplituden- und Phasenbeziehungen zwischen den Luftschall- und Körperschallsensorsignalen einstellbar oder programmierbar sind.

15. Hörsystem nach einem der vorhergehenden Ansprüche, **gekennzeichnet durch** Einheiten (16, 17, 19) zum analogen Vorverstärken und zum analogen elektronischen Weiterverarbeiten der Luftschall- und Körperschallsensorsignale.

16. Hörsystem nach einem der Ansprüche 1 bis 14, **gekennzeichnet durch** Einheiten (30, 31) zum Vorverstärken und zum Analog-Digital-Wandeln der Luftschall- und Körperschallsensorsignale.

17. Hörsystem nach Anspruch 15, **dadurch gekennzeichnet, dass** die elektronische Anordnung einen digitalen Signalprozessor (29) zum Weiterverarbeiten der digitalen Signale und/oder zum Generieren von digitalen Signalen für eine Tinnitusmaskierung aufweist.

18. Hörsystem nach Anspruch 17, **gekennzeichnet durch** eine zwischen den Signalprozessor (29) und die ausgangsseitige aktorische Anordnung (20, 20') geschaltete Digital-Analog-Wandlereinheit (32, 32') zum Digital-Analog-Wandeln der von dem Signalprozessor abgegebenen Ansteuersignale für die ausgangsseitige aktorische Anordnung.

19. Hörsystem nach Anspruch 17 oder 18, **gekennzeichnet durch** eine vorzugsweise PC-basierte Telemetrieeinrichtung (39) zur drahtlosen Übertragung von Daten zwischen der externen Einheit (41), insbesondere einem externen Programmiersystem, und dem Signalprozessor (29).

20. Hörsystem nach Anspruch 19, **dadurch gekennzeichnet, dass** dem Signalprozessor (29) eine wiederholt beschreibbare, implantierbare Speicheranordnung (S₁, S₂) zur Aufnahme und Wiedergabe eines Betriebsprogramms zugeordnet ist und mindestens Teile des Betriebsprogramms durch von der externen Einheit (41) über die Telemetrieeinrichtung (39) übermittelte Daten geändert oder ausgetauscht werden können.

21. Hörsystem nach Anspruch 19 oder 20, **gekennzeichnet durch** eine Zwischenspeicheranordnung (S₄, S₅) zum Zwischenspeichern von von der externen Einheit (41) über die Telemetrieeinrichtung (39) übermittelten Daten vor dem Weiterleiten an den Signalprozessor (29).

22. Hörsystem nach Anspruch 21, **gekennzeichnet durch** eine Überprüfungslogik (36) zum Überprüfen von in der Zwischenspeicheranordnung (S₄, S₅) gespeicherten Daten vor dem Weiterleiten an den Signalprozessor (29).

23. Hörsystem nach einem der Ansprüche 17 bis 22, **gekennzeichnet durch** einen Mikroprozessorbaustein (36), insbesondere einen Microcontroller, zum implantatinternen Steuern der Analog-Digital-Wandlereinheit (30, 30') und/oder der Digital-Analog-Wandlereinheit (32, 32') und/oder des Signalprozessors (29) über einen Datenbus (37)

24. Hörsystem nach den Ansprüchen 22 und 23, **dadurch gekennzeichnet, dass** die Überprüfungslogik und die Zwischenspeicheranordnung (S₄, S₅) in dem Mikroprozessorbaustein (36) implementiert sind.

25. Hörsystem nach Anspruch 23 oder 24, **dadurch gekennzeichnet, dass** über den Datenbus (37) und die Telemetrieeinrichtung (39) auch Programmteile oder ganze Softwaremodule zwischen der Außenwelt, dem Mikroprozessorbaustein (36) und dem Signalprozessor (29) übermittelbar sind.

26. Hörsystem nach einem der Ansprüche 23 bis 25, **dadurch gekennzeichnet, dass** dem Mikroprozessorbaustein (36) eine implantierbare Speicheranordnung (S₃) zum Speichern eines Arbeitsprogramms für den Mikroprozessorbaustein zugeordnet ist, und mindestens Teile des Arbeitsprogramms für den Mikroprozessorbaustein durch von der externen Einheit (41) über die Telemetrieeinrichtung (39) übermittelte Daten geändert oder ausgetauscht werden können.

27. Hörsystem nach einem der Ansprüche 20 bis 26, **dadurch gekennzeichnet, dass** mindestens zwei Speicherbereiche (S₁, S₂) zur Aufnahme und Wiedergabe mindestens des Betriebsprogramms des Signalprozessors (29) vorgesehen sind.

28. Hörsystem nach einem der Ansprüche 21 bis 27, **dadurch gekennzeichnet, dass** die Zwischenspeicheranordnung mindestens zwei Speicherbereiche (S₄, S₅) zur Aufnahme und Wiedergabe von von der externen Einheit (41) über die Telemetrieeinrichtung (39) übermittelten Daten aufweist.

29. Hörsystem nach einem der Ansprüche 17 bis 28, **dadurch gekennzeichnet, dass** dem Signalprozessor (29) ferner ein vorprogrammierter, nicht überschreibbarer Festspeicherbereich (So) zugeordnet ist.

30. Hörsystem nach einem der Ansprüche 19 bis 29, **dadurch gekennzeichnet, dass** die Telemetrieeinrichtung (39) zur Übermittlung auch von Betriebsparametern zwischen dem implantierbaren Teil des Systems und der externen Einheit (41) ausgelegt ist.

31. Hörsystem nach Anspruch 14 und einem der Ansprüche 17 bis 30, **dadurch gekennzeichnet, dass** in dem Signalprozessor (29) Softwaremodule zum individuellen Einstellen oder Programmieren der Amplituden- und Phasenbeziehungen zwischen den Luftschall- und Körperschallsensorsignalen implementiert sind.

32. Hörsystem nach einem der Ansprüche 17 bis 31, **dadurch gekennzeichnet, dass** in dem Signalprozessor (29) Softwaremodule zum Konfigurieren der spektralen, zeitlichen, amplituden- und phasenbezogenen Eigenschaften der Signale der ausgangsseitigen aktorischen Anordnung (20, 20') implementiert sind.

33. Hörsystem nach einem der Ansprüche 17 bis 32, **dadurch gekennzeichnet, dass** in dem Signalprozessor (29) Softwaremodule implementiert sind, die parallel zum Hörgerätebetrieb die Maskierung eines Tinnitus ermöglichen.

34. Hörsystem nach einem der Ansprüche 17 bis 33, **dadurch gekennzeichnet, dass** in dem Signalprozessor (29) ein Softwaremodul zum Approximieren einer optimalen Stimulation auf der Basis eines lernfähigen neuronalen Netzwerkes implementiert ist.

35. Hörsystem nach einem der Ansprüche 31 bis 34, **dadurch gekennzeichnet, dass** die Softwaremodule aufgrund wissenschaftlicher Erkenntnisse einmalig in einem Programmspeicher (S₁, S₂) des digitalen Signalprozessors (29) abgelegt sind und unverändert bleiben.

36. Hörsystem nach einem der Ansprüche 31 bis 34, **dadurch gekennzeichnet, dass** die Softwaremodule dynamisch, also lernfähig ausgelegt sind.

37. Hörsystem nach Anspruch 36, **dadurch gekennzeichnet, dass** die Softwaremodule für eine Parameteranpassung durch "Training" durch den Implantatträger und/oder unter Zuhilfenahme weiterer externer Hilfsmittel adaptiv ausgelegt sind.

38. Hörsystem nach einem der vorhergehenden Ansprüche, **dadurch gekennzeichnet, dass** die ausgangsseitige aktorische Anordnung (20, 20') mindestens einen elektromechanischen Wandler (60, 81, 91) zum mechanischen Stimulieren des Mittel- oder Innenohres und/oder intracochleäre Reizelektroden (70, 92) zum Stimulieren des Innenohres durch elektrische Reizung aufweist.

39. Hörsystem nach einem der vorhergehenden Ansprüche, **gekennzeichnet durch** ein implantatseitiges sekundäres elektrisches Speicherelement (42) und eine externe Ladevorrichtung (63, 64) zum drahtlosen, transkutanen Nachladen dieses Speicherelements.

## Claims

1. A totally implantable hearing system for rehabilitation of hearing disorders, comprising at least one sensor (14, 14') which is adapted to pick up at least airborne sound and to convert it into electrical signals, an electronic module (18, 28) including an electronic means for audio signal processing and amplification, an output-side actuator arrangement (20, 20') for stimulation of the middle or inner ear, and an electrical power supply unit (42), **characterized in that** the hearing system comprises at least one further implantable sensor (15, 15') which is adapted to pick up at least body sound-induced signals and to convert them into electrical signals, and **in that** the ratio of said airborne sound signals and said body sound signals is individually adjustable by electronic computation within said electronic means of said airborne sound sensor signals and said body sound sensor signals.

2. The hearing system of claim 1, **characterized in that** the at least one body sound sensor (15, 15') operates in accordance with the electromagnetic, electrodynamic, piezoelectric, magnetostrictive or dielectric (capacitive) transducer principle.

3. The hearing system of claim 2, **characterized in that** the at least one body sound sensor (15, 15') is an on-chip semiconductor transducer.

4. The hearing system of any one of claims 1 to 3, **characterized in that** the body sound sensor (15, 15') comprises a mechanical/electrical transducer (45) which is coupled to an inert mass (47) suspended for oscillating within a sensor housing (46).

5. The hearing system of any one of the preceding claims, **characterized in that** the transmission range of the at least one body sound sensor (15, 15') is from 100 Hz to 10 kHz.

6. The hearing system of any one of the preceding claims, **characterized in that** the at least one body sound sensor (15, 15') is tuned such that its first mechanical resonant frequency is at the upper spectral end of the transmission frequency range.

7. The hearing system of any one of the preceding claims, **characterized in that** the at least one body sound sensor (15, 15') is designed to be hermetically sealed.

8. The hearing system of any one of the preceding claims, **characterized in that** the at least one body sound sensor (15, 15') is implantable separate from the electronic module (18, 28), and preferably is connected to the electronic module (18, 28) via a detachable plug connection (66).

9. The hearing system of any one of claims 1 to 7, **characterized in that** the body sound sensor (15, 15') is integrated into the electronic module (18, 28) of the implant system.

10. The hearing system of any one of the preceding claims, **characterized in that** the ratio of airborne sound signals to body sound signals is adjustable via a wireless telemetry means by which data can be transmitted between an external unit and the implant.

11. The hearing system of any one of the preceding claims, **characterized in that** it is designed as a binaural system for rehabilitation of a hearing disorder of both ears comprising two system units, each of which is associated to one of the two ears, wherein at least one of the two system units is provided with a body sound sensor (15, 15').

12. The hearing system of any one of the preceding claims, **characterized in that** the at least one airborne sound sensor (14, 14') is adapted and implantable for picking up a mixture of airborne sound and body sound and for converting it into corresponding electrical signals.

13. The hearing system of any one of the preceding claims, **characterized in that** the at least one body sound sensor (15, 15') is adapted and implantable for picking up substantially body sound only and for converting it into corresponding electrical signals.

14. The hearing system of any one of the preceding claims, **characterized in that** the amplitude and phase relationships between the airborne sound sensor signals and the body sound sensor signals are adjustable or programmable.

15. The hearing system of any one of the preceding claims, **characterized by** units (16, 17, 19) for analog preamplification and analog electronic further processing of the airborne sound sensor signals and the body sound sensor signals.

16. The hearing system of any one of claims 1 to 14, **characterized by** units (30, 31) for preamplification and for analog-to-digital conversion of the airborne sound sensor signals and the body sound sensor signals.

17. The hearing system of claim 15, **characterized in that** the electronic means comprises a digital signal processor (29) for further processing of the digital signals and/or for generating digital signals for tinnitus masking.

18. The hearing system of claim 17, **characterized by** a digital-to-analog converter unit (32, 32') connected between the signal processor (29) and the output-side actuator arrangement (20, 20') for digital-to-analog conversion of the control signals for the output-side actuator arrangement which are provided by the signal processor.

19. The hearing system of claim 17 or 18, **characterized by** a, preferably PC-based, wireless telemetry means (39) for wireless transmission of data between the external unit (41), particularly an external programming system, and the signal processor (29).

20. The hearing system of claim 19, **characterized in that** a repeatedly rewritable implantable storage arrangement (S₁, S₂) is assigned to the signal processor (29) for storage and retrieval of an operating program, and **in that** at least parts of the operating program can be changed or replaced by data transmitted from the external unit (41) via the telemetry means (39).

21. The hearing system of claim 19 or 20, **characterized by** a buffer storage arrangement (S₄, S₅) for buffering data transmitted from the external unit (41) via the telemetry means (39) before being relayed to the signal processor (29).

22. The hearing system of claim 21, **characterized by** a checking logic (36) for checking data stored in the buffer storage arrangement (S₄, S₅) before said data is relayed to the signal processor (29).

23. The hearing system of any one of claims 17 to 22, **characterized by** a microprocessor module (36), particularly a micro controller, for controlling internally in the implant the analog-digital converter unit (30, 30') and/or said digital-analog converter unit (32, 32') and/or said signal processor (29) via a data bus (37).

24. The hearing system of claims 22 and 23, **characterized in that** the checking logic and the buffer storage arrangement (S₄, S₅) are implemented in the microprocessor module (36).

25. The hearing system of claim 23 or 24, **characterized in that**, via the data bus (37) and the telemetry means (39), also program parts or complete software modules can be transmitted between the external world, the microprocessor module (36) and the signal processor (29).

26. The hearing system of any one of claims 23 to 25, **characterized in that** an implantable storage arrangement (S₃) for storage of an operating program for the microprocessor module is assigned to the microprocessor module (36), and at least parts of the operating program for the microprocessor module can be changed or replaced by data transmitted from the external unit (41) via the telemetry means (39).

27. The hearing system of any one of claims 20 to 26, **characterized by** at least two storage areas (S₁, S₂) for storage and retrieval of at least said operating program of the signal processor (29).

28. The hearing system of any one of claims 21 to 27, **characterized in that** the buffer storage arrangement comprises at least two storage areas (S₄, S₅) for storage and retrieval of data transmitted from the external unit (41) via the telemetry means (39).

29. The hearing system of any one of claims 17 to 28, **characterized in that** a preprogrammed read-only memory area (So) is assigned to the signal processor (29).

30. The hearing system of any one of claims 19 to 29, **characterized in that** the telemetry means (39) is adapted for transmission of operating parameters between the implantable part of the system and the external unit (41).

31. The hearing system of claim 14 and any one of claims 17 to 30, **characterized in that** software modules for individually controlling and programming of said amplitude and phase relationships between the airborne sound signals and the body sound signals are implemented in the signal processor (29).

32. The hearing system of any one of claims 17 to 31, **characterized in that** software modules for configuring the spectral, temporal amplitude- and phase-referenced properties of the signals of the output-side actuator arrangement (20, 20') are implemented in the signal processor (29).

33. The hearing system of any one of claims 17 to 32, **characterized in that** software modules are implemented in the signal processor (29) which enable tinnitus masking concurrent with the hearing device operation.

34. The hearing system of any one of claims 17 to 33, **characterized in that** a software module for approximation of an optimized stimulation is implemented in the signal processor (29) based on an adaptive neural network.

35. The hearing system of any one of claims 31 to 34, **characterized in that** the software modules, based upon scientific findings, are stored once in a program storage (S₁, S₂) of the digital signal processor (29) and remain unchanged.

36. The hearing system of any one of claims 31 to 34, **characterized in that** the software modules are designed to be dynamic, that is adaptive.

37. The hearing system of claim 36, **characterized in that** the software modules are designed to be adaptive for parameter matching by "training" by the implant wearer and/or by assistance of further external aids.

38. The hearing system of any one of the preceding claims, **characterized in that** said output-side actuator arrangement (20, 20') comprises at least one electromechanical transducer (60, 81, 91) for mechanical stimulation of the middle ear or the inner ear and/or intracochlear stimulation electrodes (70, 92) for electrical stimulation of the inner ear.

39. The hearing system of any one of the preceding claims, **characterized by** an implant-side secondary electrical storage element (42) and an external charging device (63, 64) for wireless transcutaneous charging of such storage element.

## Revendications

1. Système auditif pouvant être implanté complètement pour la rééducation de troubles auditifs comprenant au moins un capteur (14, 14'), qui est conçu de telle sorte qu'il reçoit au moins les bruits aériens et les convertit en signaux électriques, un module électronique (18, 28) comportant un dispositif électronique pour le traitement et l'amplification de signal audio, un dispositif (20, 20') acteur agencé côté sortie pour la stimulation de l'oreille moyenne ou de l'oreille interne ainsi qu'une unité électrique d'alimentation en énergie (42), **caractérisé en ce que** le système auditif présente au moins un autre capteur (15, 15') implantable, qui est conçu de telle sorte qu'il reçoit au moins des signaux induits par des bruits de structure et les convertit en signaux électriques, et **en ce que** le rapport entre les signaux des bruits aériens et les signaux des bruits de structure peut être réglé individuellement par le calcul électronique des signaux du capteur de bruits aériens et des signaux du capteur de bruits de structure dans le dispositif électronique.

2. Système auditif selon la revendication 1, **caractérisé en ce que** l'au moins un capteur de bruits de structure (15, 15') travaille selon le principe du convertisseur électromagnétique, électrodynamique, piézoélectrique électrique électrique, magnétostrictif ou diélectrique (capacitif).

3. Système auditif selon la revendication 2, **caractérisé en ce que** l'au moins un capteur de bruits de structure (15, 15') est conçu comme un convertisseur à semi-conducteurs sur puce.

4. Système auditif selon l'une des revendications 1 à 3, **caractérisé en ce que** le capteur de bruits de structure (15, 15') présente un convertisseur (45) mécanoélectrique, qui est couplé à une masse (47) inerte, suspendue de façon vibrante dans un boîtier de capteur (46).

5. Système auditif selon l'une des revendications précédentes, **caractérisé en ce que** la plage de transmission du au moins un capteur de bruits de structure (15, 15') se situe entre 100 Hz et 10 kHz.

6. Système auditif selon l'une des revendications précédentes, **caractérisé en ce que** l'au moins un capteur de bruits de structure (15, 15') est adapté de telle sorte que sa première fréquence de résonance mécanique se situe à l'extrémité spectrale supérieure de la plage de fréquences de transmission.

7. Système auditif selon l'une des revendications précédentes, **caractérisé en ce que** l'au moins un capteur de bruits de structure (15, 15') est structuré de façon hermétiquement étanche.

8. Système auditif selon l'une des revendications précédentes, **caractérisé en ce que** l'au moins un capteur de bruits de structure (15, 15') peut être implanté séparément du module électronique (18, 28) et raccordé de préférence au module électronique (18, 28) au moyen d'une connexion à fiche (66) amovible.

9. Système auditif selon l'une des revendications 1 à 7, **caractérisé en ce que** le capteur de bruits de structure (15, 15') est intégré dans le module électronique (18, 28) du système d'implant.

10. Système auditif selon l'une des revendications précédentes, **caractérisé en ce que** le rapport entre les signaux de bruits aériens et les signaux de bruits de structure peut être réglé au moyen d'un appareil de télémétrie sans fil, au moyen duquel des données peuvent être transmises entre une unité externe et l'implant.

11. Système auditif selon l'une des revendications précédentes, **caractérisé en ce qu'**il est conçu comme système binaural pour la rééducation d'un trouble auditif des deux oreilles, qui présente deux unités de système qui sont attribuées chacune à l'une des deux oreilles, au moins l'une des deux unités du système étant pourvue d'un capteur de bruits de structure (15, 15').

12. Système auditif selon l'une des revendications précédentes, **caractérisé en ce que** l'au moins un capteur de bruits aériens (14, 14') est conçu et peut être implanté de telle sorte qu'il reçoit un mélange de bruits aériens et de bruits de structure et les convertit en signaux électriques appropriés.

13. Système auditif selon l'une des revendications précédentes, **caractérisé en ce que** l'au moins un capteur de bruits de structure (15, 15') est conçu et peut être implanté de telle sorte qu'il reçoit essentiellement seulement des signaux de bruits de structure et les convertit en signaux électriques correspondants.

14. Système auditif selon l'une des revendications précédentes, **caractérisé en ce que** les rapports d'amplitude et rapports de phase entre les signaux de bruits aériens et les signaux de bruits de structure sont réglables ou programmables.

15. Système auditif selon l'une des revendications précédentes, **caractérisé par** des unités (16, 17, 19) pour la préamplification analogique et pour le retraitement électronique analogique des signaux de bruits aériens et des signaux de bruits de structure.

16. Système auditif selon l'une des revendications 1 à 14, **caractérisé par** des unités (30, 31) pour la préamplification et pour la conversion analogique-numérique des signaux de bruits aériens et des signaux de bruits de structure.

17. Système auditif selon la revendication 15, **caractérisé en ce que** le dispositif électronique présente un processeur de signal (29) numérique pour le retraitement des signaux numériques et/ou pour la génération de signaux numériques pour un masquage de tinnitus.

18. Système auditif selon la revendication 17, **caractérisé par** une unité de convertisseur numérique-analogique (32, 32') branchée entre le processeur de signal (29) et le dispositif (20, 20') acteur agencé côté sortie pour la conversion numérique-analogique des signaux de commande émis par le signal de processeur pour le dispositif acteur agencé côté sortie.

19. Système auditif selon la revendication 17 ou 18, **caractérisé par** un appareil de télémétrie (39) de préférence basé sur PC pour la transmission sans fil de données entre l'unité (41) externe, en particulier un système de programmation externe, et le processeur de signal (29).

20. Système auditif selon la revendication 19, **caractérisé en ce qu'**un dispositif de mémoire (S₁, S₂) pouvant être décrit de façon répétée et pouvant être implanté pour l'enregistrement et la restitution d'un programme de service est attribué au processeur de signal (29) et au moins des parties du programme de service peuvent être modifiées ou remplacées par des données transmises par l'unité (41) externe au moyen de l'appareil de télémétrie (39).

21. Système auditif selon la revendication 19 ou 20, **caractérisé par** un dispositif de mémoire intermédiaire (S₄, S₅) pour le stockage intermédiaire de données transmises à partir de l'unité (41) externe par l'appareil de télémétrie (39) avant la transmission au processeur de signal (29).

22. Système auditif selon la revendication 21, **caractérisé par** une logique de contrôle (36) pour le contrôle de données stockées dans le dispositif de mémoire intermédiaire (S₄, S₅) avant la transmission au processeur de signal (29).

23. Système auditif selon l'une des revendications 17 à 22, **caractérisé par** un module de microprocesseur (36), en particulier un microcontrôleur, pour la commande interne à l'implant de l'unité de convertisseur analogique-numérique (30, 30') et/ou de l'unité de convertisseur numérique-analogique (32, 32') et/ou du processeur de signal (29) au moyen d'un bus de données (37).

24. Système auditif selon les revendications 22 et 23, **caractérisé en ce que** la logique de contrôle et le dispositif de mémoire intermédiaire (S₄, S₅) sont implantés dans le module de microprocesseur (36).

25. Système auditif selon la revendication 23 ou 24, **caractérisé en ce que** des parties de programme ou des modules de logiciel complets peuvent également être transmis entre le monde extérieur, le module de microprocesseur (36) et le processeur de signal (29) au moyen du bus de données (37) et de l'appareil de télémétrie (39).

26. Système auditif selon l'une quelconque des revendications 23 à 25, **caractérisé en ce qu'**un dispositif de mémoire (S₃) implantable pour le stockage d'un programme de travail pour le module du microprocesseur est attribué au module du microprocesseur (36), et au moins des parties du programme de travail pour le module de microprocesseur peuvent être modifiées ou remplacées par des données transmises à partir de l'unité (41) externe par l'appareil de télémétrie (39).

27. Système auditif selon l'une quelconque des revendications 20 à 26, **caractérisé en ce qu'**au moins deux zones de mémoire (S₁, S₂) sont prévues pour la réception et la restitution au moins du programme de service du processeur de signal (29).

28. Système auditif selon l'une des revendications 21 à 27, **caractérisé en ce que** le dispositif de mémoire intermédiaire présente au moins deux zones de mémoire (S₄, S₅) pour la réception et la restitution de données transmises à partir de l'unité (41) externe par l'appareil de télémétrie (39).

29. Système auditif selon l'une des revendications 17 à 28, **caractérisé en ce qu'**une plage de mémoire fixe (S₀) préprogrammée et non écrasable est attribuée également au processeur de signal (29).

30. Système auditif selon l'une des revendications 19 à 29, **caractérisé en ce que** l'appareil de télémétrie (39) est conçu pour la transmission également de paramètres de service entre la partie implantable du système et l'unité (41) externe.

31. Système auditif selon la revendication 14 et l'une des revendications 17 à 30, **caractérisé en ce que** des modules de logiciel pour le réglage individuel ou la programmation des rapports d'amplitude et de phase entre les signaux de bruits aériens et les signaux de bruits de structure sont implantés dans le processeur de signal (29).

32. Système auditif selon l'une des revendications 17 à 31, **caractérisé en ce que** des modules de logiciel pour la configuration des propriétés spectrales, temporelles, spécifiques aux amplitudes et aux phases des signaux du dispositif (20, 20') acteur agencé côté sortie sont implantés dans le processeur de signal (29).

33. Système auditif selon l'une des revendications 17 à 32, **caractérisé en ce que** des modules de logiciel sont implantés dans le processeur de signal (29), lesquels modules permettent le masquage d'un tinnitus parallèlement à l'exploitation des appareils auditifs.

34. Système auditif selon l'une des revendications 17 à 33, **caractérisé en ce qu'**un module de logiciel pour l'approximation d'une stimulation optimale sur la base d'un réseau neuronal et apte à l'apprentissage est implanté dans le processeur de signal (29).

35. Système auditif selon l'une des revendications 31 à 34, **caractérisé en ce que** les modules de logiciel sont déposés sur la base de connaissances scientifiques une fois dans une mémoire de programme (S₁, S₂) du processeur de signal (29) numérique et restent inchangés.

36. Système auditif selon l'une des revendications 31 à 34, **caractérisé en ce que** les modules de logiciel sont conçus de façon dynamique, donc aptes à l'apprentissage.

37. Système auditif selon la revendication 36, **caractérisé en ce que** les modules de logiciel sont conçus de façon adaptative pour un ajustement des paramètres par "entraînement" par le porteur d'implant et/ou en recourant à d'autres moyens auxiliaires externes.

38. Système auditif selon l'une des revendications précédentes, **caractérisé en ce que** le dispositif (20, 20') acteur agencé côté sortie présente au moins un convertisseur (60, 81, 91) électromécanique pour la stimulation mécanique de l'oreille moyenne ou de l'oreille interne et/ou des électrodes d'excitation intracochléaires (70, 92) pour la stimulation de l'oreille interne par une stimulation électrique.

39. Système auditif selon l'une des revendications précédentes, **caractérisé par** un élément de mémoire (42) électrique secondaire agencé côté implant et un dispositif de chargement (63, 64) externe pour le rechargement sans fil et transcutané de cet élément de mémoire.
